(19) Europäisches Patentamt
European Patent Office
Office européen des brevets

(11) **EP 4 110 278 B1**

(12) **EUROPEAN PATENT SPECIFICATION**

(45) Date of publication and mention of the grant of the patent:
**01.07.2026 Bulletin 2026/27**

(21) Application number: **21706605.9**

(22) Date of filing: **24.02.2021**

(51) International Patent Classification (IPC):
***A61K 8/36*** (2006.01) ***A61K 8/9789*** (2017.01)
***A61Q 19/08*** (2006.01)

(52) Cooperative Patent Classification (CPC):
**A61K 8/36; A61K 8/9789; A61Q 19/08**

(86) International application number:
**PCT/EP2021/054506**

(87) International publication number:
**WO 2021/170618 (02.09.2021 Gazette 2021/35)**

# (54) COMPOSITIONS COMPRISING LUFFA CYLINDRICA ROOT EXTRACT

ZUSAMMENSETZUNGEN MIT LUFFA-CYLINDRICA-WURZELEXTRAKT

COMPOSITIONS COMPRENANT UN EXTRAIT DE RACINE CYLINDRICA LUFFA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.02.2020 EP 20305178**

(43) Date of publication of application:
**04.01.2023 Bulletin 2023/01**

(73) Proprietors:
- **Clariant International Ltd**
  **4132 Muttenz (CH)**
- **Plant Advanced Technologies PAT SA**
  **54500 Vandoeuvre-lès-Nancy (FR)**

(72) Inventors:
- **FRECHET, Mathilde**
  **31000 Toulouse (FR)**
- **CHAJRA, Hanane**
  **31170 Tournefeuille (FR)**
- **GUILLAUMIN, Agnès**
  **54170 GERMINY (FR)**

(74) Representative: **IPAZ**
**43 boulevard de Strasbourg**
**75010 Paris (FR)**

(56) References cited:

| | |
|---|---|
| **EP-A2- 0 359 196** | **JP-A- H06 157 280** |
| **JP-A- S62 226 912** | **JP-B2- 4 716 692** |
| **JP-B2- 5 946 717** | |

- **CHO H J ET AL: "Formation of bryonolic acid in cucurbitaceous plants and their cell cultures", PHYTOCHEMISTRY, ELSEVIER, AMSTERDAM, NL, vol. 31, no. 11, 1 January 1992 (1992-01-01), pages 3893 - 3896, XP026632428, ISSN: 0031-9422, [retrieved on 19920101], DOI: 10.1016/S0031-9422(00)97548-4**
- **CHEN ZHE ET AL: "The diversified function and potential therapy of ectopic olfactory receptors in non-olfactory tissues", JOURNAL OF CELLULAR PHYSIOLOGY, vol. 233, no. 3, 1 March 2018 (2018-03-01), US, pages 2104 - 2115, XP055802268, ISSN: 0021-9541, DOI: 10.1002/jcp.25929**
- **LEE SUNG-JOON ET AL: "Therapeutic potential of ectopic olfactory and taste receptors", NATURE REVIEWS DRUG DISCOVERY, NATURE PUBLISHING GROUP, GB, vol. 18, no. 2, 30 November 2018 (2018-11-30), pages 116 - 138, XP036688583, ISSN: 1474-1776, [retrieved on 20181130], DOI: 10.1038/S41573-018-0002-3**

EP 4 110 278 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the invention

The present invention refers to a root extract of plant *Luffa cylindrica,* enriched in bryonolic acid, a method for preparing the enriched root extract and cosmetic or dermatological or nutraceutical compositions comprising, as an active ingredient, the enriched root extract. Another subject-matter of the invention relates to the use of said enriched root extract, or compositions comprising said enriched root extract as an active ingredient, for restoring the dysregulated bioenergetic functions (stimulating skin energetic metabolism), for restoring cell viability even in fatigue cells, for preventing stress induced by toxic agents, for preventing oxidative stress, more generally for preventing and/or delaying the sign of aging and for energizing the skin cells, for regulating the skin ectopic olfactory receptors (ORs) expression involved in the immune response, the skin inflammation, the skin neuronal physiology, the skin metabolic modulation, the wound healing and the skin stress responses.

Background of the invention

The skin is the human body's first barrier. It protects the organs from temperature and humidity differences and from attack by the external environment, such as UV-rays or pollutant agents. However, excessive chemical and physical stimulations (exposure to sunlight, to light or to UV; stress and malnutrition) deteriorate the normal functions of the skin and induce aging thereof. Thus, it is important to maintain skin renewal and repair by stimulating the metabolism of the cells which constitute it and notably the cellular energy metabolism. Specifically, in cells, energy is used, *inter alia,* for manufacturing new proteins, supplying nutrients, expelling cellular waste and repairing DNA damage.

Bioenergetic processes functionality is crucial to maintain a good performance of all organs including skin. At the cellular level, three key metabolic pathways: glycolysis, pentose-phosphate pathway and tricarboxylic acid (TCA) cycle are interlinked and constitute the first stages of cell metabolism (Respiratory metabolism: glycolysis, the TCA cycle and mitochondrial electron transport, Current Opinion in Plant Biology (2004*)).*

Glycolysis describes the metabolic pathway that converts glucose into pyruvate and a hydrogen ion. The free energy released in this process is used to form the high-energy molecules ATP (adenosine triphosphate) and NADH (reduced nicotinamide adenine dinucleotide). NADH plays a key role in energy metabolism by accepting and donating electron. TCA cycle occuring in the matrix of mitochondria consists of a number of reactions, which generate NADH and the reduced flavin adenin dinucleotide ($FADH_2$), which can in turn be used by the oxidative phosphorylation pathway to generate ATP. As NADH, FADH2 is also considered to be a high energy molecule for cell metabolism. Indeed, NADH and FADH2 can donate their electrons to the electron carriers of the electron transport chain to drive oxidative phosphorylation responsible for ATP production. Additionally, TCA cycle is central to many pathways of metabolism, and therefore there must always be a large supply of the intermediates required to its activity. For example, oxaloacetate, which can be synthesized from pyruvate previously produced during glycolysis process, is the direct precursor of the amino acid aspartate. This amino acid is important for protein synthesis. Pyruvate from glycolysis enters the TCA cycle through the action of the pyruvate dehydrogenase complex. The pentose phosphate pathway, which took place in cytosol, oxidizes glucose to make NADPH and other carbohydrates for biosynthesis.

As detailed above, these three pathways generate metabolites required for mitochondrial ATP production by the oxidative phosphorylation process. Mitochondria is the indispensable organite in the center of this process. The whole of these series of energy transformations is called cellular respiration and its well functionality warrants skin cell health.

Actually, while glycolysis takes place in the cytoplasm, TCA cycle happens in the mitochondria. Therefore solutes and metabolites coming from glycolysis have to intransit through mitochondrial membrane in order to be taken over the TCA cycle.

This role is ensured by SLC25, which is a specific family of transporters aiming to shuttle a variety of metabolites across the mitochondrial membrane.

It has been demonstrated that defects of these SLC25 mitochondrial carriers that supply mitochondria with the substrates of oxidative phosphorylation, inorganic phosphate and ADP, are responsible for diseases characterized by defective energy production (Diseases caused by defects of mitochondrial carriers: A review, Biochimica et Biophysica Acta, 2008*).* Mitochondrial transporters of the SLC25 family and associated diseases: a review, J Inherit Metab Dis. 2014 Jul;37(4):565-75. 2014*).* Among them, SLC25A12, a malate-aspartate shuttle, is described as being responsible for the mitochondrial transportation of NADH produced during glycolysis into the mitochondria to supply TCA cycle.

Therefore, SLC25A12 plays a crucial role to link glycolysis with TCA and oxidative phosphorylation metabolic pathways.

Environmental damages and stress accumulation lead to depletion of energy metabolism, impacting overall metabolism *(*Reprogramming of energy metabolism as a driver of aging, Oncotarget. (2016*)).* In addition, mitochondria dysfunction has been described in several diseases and is highly associated to aging *(*López-Otín, et al. 2013, Daniel

Schniertshauer, et al. Age-Dependent Loss of Mitochondrial Function in Epithelial Tissue Can Be Reversed by Coenzyme Q10, J Aging Res, 2018*)*. This dysfunction results from alterations induced from exogenous harmful stresses (UV rays, pollution...) but also by endogenous reactive oxygen species (ROS) produced during oxidative phosphorylation process itself. In young and metabolically "healthy" cells, anti-oxidant systems ensure the management of an acceptable ROS amount to maintain an efficient cellular respiration and production of ATP to react efficiently to any threat (*Bee Ling Tan et al. 2018)*. In aged cells, these systems are overwhelmed following internal and external stresses that accumulate.

Consequently, the energy necessary for the overall cellular metabolic functions is less produced, which result in a disbalanced homeostasis and abnormal protein production program. This may lead to dramatic consequences associated to characteristic aged skin signs. Especially, extracellular matrix is a complex layout of macromolecules that are synthetized by skin cells and lend firmness and elasticity to the skin. These macromolecules are produced through endoplasmic reticulum (ER) and then Golgi apparatus, where they are synthetized and matured before their excretion. SLC35 are a specific family of transporter for nucleotide sugar and adenosine 3'-phospho 5'-phosphosulfate (PAPS) into the Golgi and ER and therefore have an indispensable role in the maturation of matrix macromolecules *(*Molecular physiology and pathology of the nucleotide sugar transporter family (SLC35), Eur J Physiol, 2004*)*.

Sponge gourd, also named loofah, (*Luffa cylindrica,* Cucurbitaceae) is an annual vegetable upland crop that originates in India and Southern Asia and is distributed mainly in tropical to warm-temperate areas. In China, various parts of this plant, including flowers, fruits, seeds, leaves, and roots, are used for alleviating or reducing inflammations such as pharyngitis, rhinitis, mastitis, edema, swellings, burns, and hemorrhoids.

Bryonolic acid, a pentacyclic triterpenoid, was first isolated in 1960 from the roots of *Bryonia dioica* Jacq. (Cucurbitaceae) and later from the roots and cultured cells of *Luffa cylindrica.* Roem. Hi Jai Cho et al. (Formation of bryonolic acid in cucurbitaceous plants and their cell cultures, phytochemistry, vol. 31, No. 11, pp. 3893-3896, 1992) investigated twenty-one species or varieties of cucurbitaceous plants belonging to nine genera and their cell cultures with respect to their ability to produce bryonolic acid. Bryonolic acid was found in all investigated species except for two of them, being localized exclusively in the roots of mature plants or in the radicles of seedlings.

Some biological activities have been previously reported for bryonolic acid, including anti-allergic properties in rodents and a variety of cytotoxic and antitumor activities in multiple cancer cell lines ("Bryonolic acid production in hairy roots of Trhichosanthes kirilowii Max. var. Japonica transformed with Agrobacterium rhizogenes and its cytotoxic activity" by Tadahiro TAKEDA et al. (1994)). In her thesis, Emily Clegg Barker (The isolation and biological evaluation of anti-inflammatory and chemopreventive triterpenoid natural products, chapter 2, pp. 64-85, May 2015) showed bryonolic acid exhibiting an anti-inflammatory and anti-oxidant activities as demonstrated by its robust induction of Heme oxygenase-1 expression.

On the one hand, it is thus established that bryonolic acid exhibits interesting biological activities which can be valorized, for example, in the cosmetic domain. Additionally, bryonolic acid is exclusively present in the roots of certain Cucurbitaceae species, such as the Luffa, Cucumis and Benincasa species, more particularly *Luffa cylindrica.* Therefore, it is advantageous to be able to access to the roots of said plants in order to get this interesting compound. But, such operation involves the destruction of all or part of the plant. Moreover, the Nagoya Protocol and the International Standard (ISO 26000) provide guidelines in order to control the access to the biodiversity and for ensuring a sustainable development and social responsibility.

On the other hand, the native root extract of the above Cucurbitaceae species contains bryonolic acid, but its concentration is not sufficient for generating significant and satisfactory results, particularly in cosmetic and/or dermatological fields.

JP-3659424B2 describes a composition containing bryonolic acid from *Luffa cylindrica* root extract for use as wound healing, for promoting cell growth and for promoting skin soothing. JP-5946717B2 discloses bryonolic acid, which may be extracted from Luffa, for promoting the production of collagen type III and for promoting wound healing on the skin. Also, EP-0359196A2 discloses a topical formulation comprising *Luffa cylindrica* extract for moisturizing, for whitening, for eliminating and for preventing the formation of wrinkles. JP-S62226912A2 discloses a composition comprising an extract of culture induced root of cucurbitaceous plant produced by infection of Agrobacterium. Such extract can be used for skin moisturizing or for the improvement of fine wrinkles, however, the specification is silent about the pathways. Also, JP-2019034899A2 discloses a composition comprising fruits and stem extract of loofah, which is bryonolic acid-free, for preventing wrinkles formation by inhibiting elastase activity as a pathway.

JP-H07109214A relates to a sebum secretion promoter comprising bryonolic acid from cultured cell of cucurbitaceous plant.

JP-2009256271A provides an extract from aerial part of *Luffa cylindrica* for promoting the aquaporin expression and then to confer to the skin its capacity to be hydrated. Also, CN-107773470A discloses a face mask based on Luffa extract capable to moisturize the skin.

JP-H11240823A discloses a composition containing *Luffa cylindrica* extract for promoting hair papillae growth.

JP-4716692B2 discloses a composition having an excellent anti-inflammatory effect of suppressing erythema UV, based on ethanol loofah root extract.

KR-101256180B1 discloses a skin whitening composition containing Luffa seed extract as active ingredient capable for suppressing tyrosinase activity in melanocytes.

In spite of the availability of root extract of Cucurbitaceae species, such as Luffa, as active ingredient, there is still a need for a new cosmetic and/or dermatological and/or nutraceutical active ingredient consisting of an improved root extract of said plants, enriched in bryonolic acid. Such enriched root extract is furthermore obtained in compliance with the principles of sustainable development, social and environmental terms, meaning by preserving the plants.

There remains also a need for new cosmetic or dermatological or nutraceutical compositions comprising at least a root extract of *Luffa cylindrica* enriched in bryonolic acid as an active ingredient. Advantageously, said enriched root extract of *Luffa cylindrica,* as an active ingredient, enables to exhibit cosmetic and/or dermatological effects never obtained and described so far.

It has been surprisingly found that the amount of bryonolic acid within the root of *Luffa cylindrica* can be increased, for example, by culturing said plant under particular conditions. The inventors have demonstrated that culturing said plant under particular conditions allows the enrichment of the amount of bryonolic acid within their roots. The extract of said roots enriched in bryonolic acid can be advantageously used as an active ingredient in cosmetic and/or dermatological and/or nutraceutical fields. Moreover, biological targets and pathways highlighted and reached by the enriched root extract of *Luffa cylindrica* allow to obtain global skin beneficial effects compared to those described in the prior art. Indeed, it has been demonstrated that the root extract of *Luffa cylindrica* enriched in bryonolic acid is capable for stimulating skin energetic metabolism by modulating the mitochondrial transporters SLC25, for upregulating intrinsic anti-oxidative systems by stimulating glutathione s-transferase theta 2, glutathione peroxidase 2 and 3, for detoxyfying peroxides by enhancing peroxiredoxin-2, for restoring efficiently cell bioenergetic profiles altered by the environmental stressors, for improving the skin structure and/or for reducing wrinkles and fine lines by the stimulation of collagen types I and IV protein expression and elastic fibers.

The root extract of *Luffa cylindrica* according to the present invention as well as the root extract of *Luffa cylindrica* obtained by the method according to the present invention exhibits, unexpectedly, skin beneficial effects, specifically for fueling cells with health and giving skin rejuvenation based on the restoration of cellular and mitochondrial bioenergetics functions while stimulating endogenous anti-oxidant properties in order to enhance global cellular metabolism and ultimately stimulate extracellular matrix macromolecules. In other words, the root extract of *Luffa cylindrica* according to the present invention as well as the root extract of *Luffa cylindrica* obtained by the method according to the present invention is, for example, suitable for stimulating and/or restoring skin energetic metabolism, for restoring cell viability, even in fatigue cells, for inducing protective response of the skin against external agents (toxic agents, exposure to the sun, or UV, to light, stress, malnutrition), for preventing oxidative stress, more generally for preventing and/or delaying the signs of aging, and for improving the firminess, the tonicity and the elasticity of the skin.

## Summary of the invention

The present invention refers to a root extract of the plant *Luffa cylindrica,* wherein said root extract comprises bryonolic acid, wherein the bryonolic acid represents at least 10% by weight, relative to the total weight of the dry extract.

The present invention also refers to a method for preparing a root extract of the plant *Luffa cylindrica,* said method comprising the following steps:

a) a step of cultivating *Luffa cylindrica* under soilless conditions, in particular aeroponically,
b) a step of stimulating the roots of said plant,
c) a step of solid/liquid extraction by root maceration of the roots optionally stimulated in step b),
d) recovery of the extract obtained in step c), and
e) optionally, a step of dilution and/or clarification of the extract recovered in step d) by successive filtrations, in particular by clarifying filtration and/or sterilizing filtration.

The present invention also refers to a cosmetic or dermatological or nutraceutical composition comprising a root extract of the plant *Luffa cylindrica* as defined above, and optionally one or more excipients, which are preferably cosmetically or dermatologically or nutraceutically acceptable.

The present invention also refers to a root extract of the plant *Luffa cylindrica* as defined above or a composition comprising a root extract of the plant *Luffa cylindrica* as defined above for use in stimulating and/or restoring skin energetic metabolism, restoring cell viability (even in fatigue cells), inducing protective response of the skin against external agents, preventing oxidative stress, preventing and/or delaying the signs of aging, and/or improving the firmness, tonicity and/or elasticity of the skin.

The present invention also refers to the use of a root extract of the plant *Luffa cylindrica* as defined above or of a composition (preferably cosmetic composition) comprising a root extract of the plant *Luffa cylindrica* as defined above for stimulating and/or restoring skin energetic metabolism, restoring cell viability (even in fatigue cells), inducing protective

response of the skin against external agents, preventing oxidative stress, preventing and/or delaying the signs of aging, and/or improving the firmness, tonicity and/or elasticity of the skin.

The invention also refers to a cosmetic skin care method for preventing or delaying the appearance of skin aging effects, said method comprises applying on at least a part of the body or face skin a cosmetic composition as defined above.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

Description of figures

Fig. 1: Seahorse XF cell mitochondrial stress test profile (Bioenergetic profile)

Fig. 2 shows the Seahorse XF cell mitochondrial stress test profiles of 44-year-old fibroblasts in presence or not of a stressor (solvent), with and without the cosmetic composition comprising root extract of *Luffa cylindrica* enriched in bryonolic acid according to the invention (n=3). Respiratory profiles. NT for non-stressed cells (control fibroblasts).

Fig. 3 shows the skin morphological observation performed on formalin fixed paraffin embedded skin sections after HES (hematoxylin eosin saffron) staining. Skin explant model treated 7 days with a formula containing 0 (placebo), 0.125%, 0.5% and 1% of root extract of *Luffa cylindrica* enriched in bryonolic acid according to the invention. Dermis densification is shown by the yellow staining. Scale bars: 100 $\mu$m.

Fig. 4 shows the elastic fibers staining performed on formalin fixed paraffin embedded skin sections after modified Verhoeff's staining. Skin explant model treated 7 days with a formula containing 0 (placebo), 0.125%, 0.5% and 1% of root extract of *Luffa cylindrica* enriched in bryonolic acid according to the invention. Elastic fibers appear in dark. Nuclei are stained in blue. Scale bars: 100 $\mu$m.

Fig. 5 shows the skin biomechanical properties measured with a cutometer on face skins of aged volunteers after twice daily application of a formula comprising root extract of *Luffa cylindrica* enriched in bryonolic acid according to the invention at 1% or a placebo formula. Skin firmness (R0), skin tonicity (Ur), skin Elasticity (Ue and R6). Results are expressed relatively to day 0 and in comparison to placebo group. Statistics: ANOVA with Dunnett's comparisons. ***: $p<0.005$, **: $p<0.01$, *: $p<0.05$.

Detailed description of the invention

A first subject-matter of the present invention relates to a root extract of the plant *Luffa cylindrica,* wherein said root extract comprises bryonolic acid, wherein the bryonolic acid represents at least 10% by weight, relative to the total weight of the dry extract.

According to an embodiment of the present invention, the bryonolic acid represents at least 15%, preferably at least 20%, at least 25%, at least 30%, at least 35%, at least 40%, at least 45%, at least 50%, at least 55% or at least 60% by weight, relative to the total weight of the dry extract.

According to one embodiment of the present invention, said root extract is in liquid form and comprises a maceration solvent selected from isopropyl myristate, triglycerides, tri-ethyl citrate, dicaprylyl ether, glyceryl isostearate, glyceryl stearate, ethyl acetate, vegetable oils or a mixture thereof. Preferably, the solvent is dicaprylyl ether. More preferably the maceration solvent is bio-based dicaprylyl ether. Such a liquid root extract corresponds to a raw liquid extract obtained after a step a) of cultivating Luffa cylindrica under soilless conditions, in particular aeroponically, an optional step b) of stimulating the roots of said plant, a step c) of solid/liquid extraction by root maceration of the roots in the optionally stimulated in step b), and a step d) of recovery of the extract obtained in step c). A "maceration solvent" is thus a solvent used for obtaining the root extract. Such solvents need to be selected from particular solvents in order to reach the desired content of bryonolic acid of at least 10% by weight, relative to the total weight of the dry extract.

According to one embodiment of the invention, said root extrat may be in a solid or in a sticky form after a further step e) of drying the root extract in a liquid form, the drying being conducted according to any method known in the art, for example for placing the root extract in liquid form in a hot and dry atmosphere in order to evaporate the maceration solvent.

The root extract in solid or sticky form may further be diluted in a dilution solvent to obtain another type of root extract in liquid form. A "dilution solvent" is thus a solvent used for diluting an already obtained root extract in solid or sticky form. The dilution solvent may notably be selected from alcohols, glycols, ethyl lactate, isopropyl myristate, triglycerides,

tri-ethyl citrate, dicaprylyl ether, glyceryl isostearate, glyceryl stearate, ethyl acetate, vegetable oils or a mixture thereof. The alcohols are preferably selected from methanol and ethanol. Indeed, while specific solvents need to be used as maceration solvents in order to reach the desired content of bryonolic acid of at least 10% by weight, relative to the total weight of the dry extract, other solvents may be used for diluting an already obtained root extract in solid or sticky form before use of the extract. In particular, the dilution solvent does not need to be the same as the maceration solvent used for obtained the extract. In the context of dilution solvents, the alcohols are preferably selected from methanol and ethanol. The glycols are preferably selected from pentylene glycol and glycerol. Preferably, the dilution solvent is selected from pentylene glycol and dicaprylyl ether. In a particular embodiment, the dicaprylyl ether is bio-based. More preferably, the dilution solvent is pentylene glycol. More preferably, the dilution solvent is dicaprylyl ether. Particularly preferably, the dilution solvent is bio-based dicaprylyl ether.

Depending if the root extract in liquid form is a raw liquid root extract in maceration solvent or a dried root extract diluted in a dilution solvent, a root extract in liquid form may comprise a maceration solvent as disclosed above or a dilution solvent as disclosed above.

By "root extract of plant" is meant the product obtained by the extraction of enriched roots of the plant *Luffa cylindrica,* said root extract being preferably, but not limited thereto, in liquid form.

By "root enriched in bryonolic acid" and "enriched root" are meant a root extract of said plant containing a higher amount of bryonolic acid, in comparison to a corresponding root extract of the same plant that can be found in nature. In the other words, by "root extract enriched in bryonolic acid" is meant a root extract comprising bryonolic acid which represents at least 10% by weight, relative to the total weight of the dry extract.

By "bryonolic acid" is meant a pentacyclic triterpenoid compound having the following general formula (I):

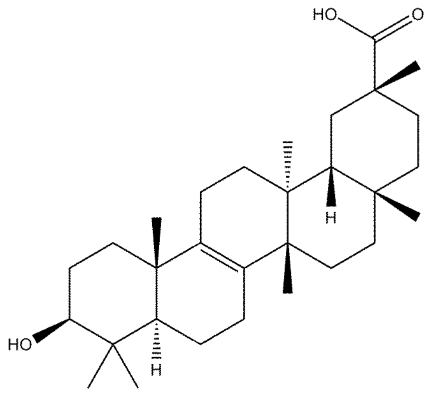

Formula (I): Bryonolic acid structure

By "dry extract" is meant the root extract of the plant which can be obtained by implementing the process according to the invention, followed by a step of drying the extract obtained, the drying being implemented according to any method well known by a person skilled in the art, in particular placing the root extract in a hot and dry atmosphere or placing the root extract on a heated plate in order to evaporate the solvent.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided

herein is intended merely to better illustrate the invention and does not pose a limitation on the scope of the invention otherwise claimed. No language in the specification should be construed as indicating any non-claimed element essential to the practice of the invention.

Unless defined otherwise, all technical and scientific terms used herein have the same meaning as commonly understood by a person skilled in the art. Although the methods and materials described herein are preferred, other methods and materials similar or equivalent to those described herein can be used in the practice or testing of the present invention as well.

Preferably, for obtaining the root extract of the plant *Luffa cylindrica* according to the present invention, when the roots are considered sufficiently developed, they are contacted with an extraction solvent by immersion or preferably by maceration, and then said extraction solvent is recovered and treated to extract secondary metabolites, comprising bryonolic acid, therefrom which have been released by the roots of said plant. This preferred method is adapted from the method developed by the company named Plant Advanced Technologies (PAT) as "PAT Plantes à Traire®" and described in the international application WO-01/33942. The teaching of this international application is incorporated by reference in the specification of the present invention.

A second subject-matter of the present invention refers to a method for preparing a root extract of the plant *Luffa cylindrica,* said method comprising the following steps:

a) a step of cultivating *Luffa cylindrica* under soilless conditions, in particular aeroponically,
b) optionally a step of stimulating the roots of said plant,
c) a step of solid/liquid extraction by root maceration of the roots optionally stimulated in step b),
d) recovery of the extract obtained in step c), and
e) optionally, a step of dilution and/or clarification of the extract recovered in step d) by successive filtrations, in particular by clarifying filtration and/or sterilizing filtration

This method is the preferred method for preparing the root extract of the plant *Luffa cylindrica* according to the present invention. In a preferred embodiment of the present invention, the root extract of the plant *Luffa cylindrica* according to the present invention is obtained by the method of the present invention. In a preferred embodiment of the present invention, the method of the present invention is used to prepare the root extract of the plant *Luffa cylindrica* according to the present invention.

In the context of the invention, the term "cultivation of plants under soilless conditions" means any mode of cultivation in which the development of plant roots does not take place in the soil. More precisely, soilless cultivation is a cultivation-type in which the roots of the plants grow in a reconstituted medium, detached from the soil. This cultivation medium is regularly irrigated with well-known nutrient solutions suitable for the cultivated plant.

Various soilless cultivation techniques exist, such as substrate-free systems which require an oxygen-enriched nutrient solution, and substrate-based systems. Among the substrate-free systems are aquaculture, for which the nutrient solution is non-circulating and is contained in a cultivation tank, the Nutrient Film Technique (NFT) for which the nutrient solution becomes enriched in dissolved oxygen in the course of its movement by exchange with air, and aeroponics, for which the roots of the plants are not in contact neither with a solid medium, nor with a liquid medium. Indeed, roots are fed by a nutrient mist obtained by misting (via a mister) the nutrient solution in a closed medium. Among the substrate-based systems is subirrigation, in which the nutrient solution penetrates the substrate via its lower part, and percolation, in which the nutrient solution is distributed by discontinuous irrigation at the upper surface of the system and then percolates to the bottom of the substrate. The mineral or organic substrate is neutral and inert, like sand, clay or rock wool, for example. This substrate may also be of synthetic origin.

The term "aeroponic plant cultivation" refers to a mode of soilless cultivation for which the roots of the plants are not in permanent contact neither with a solid medium nor with a liquid nutrient medium.

The term "nutrient solution" refers to a solution comprising essential mineral salts (nitrogen - N, phosphorus - P, potassium - K), in an optimal amount and in an optimal ratio each other to obtain maximum root growth and a maximum production of secondary metabolites.

The term "stimulating nutrient solution" refers to a nitrogen-deficient solution comprising essential mineral salts (nitrogen - N, phosphorus - P, potassium - K), in optimal amount to obtain maximum root growth and a maximum production of secondary metabolites, particularly bryonolic acid.

According to an embodiment of the method of the present invention, the plant may be fed with a nutrient solution mist obtained by misting, via a mister, of the nutrient solution in a closed medium.

According to an embodiment of the method of the present invention, the plant may be placed on trays with the aerial part of the plant above the tray and the root part below, the trays are placed on tables forming a retention area to collect the excess of a liquid diffused toward the plants, and the trays are transferred onto the tables at various stations. Teachings about this technology suitable for the aeroponic plant culture is more detailed in the international application WO2018054704A1 which is also incorporated by reference in the specification of the present invention.

According to an embodiment of the method of the present invention, during step a), the aeroponically cultivated plants are fed by spraying the roots with a nutrient solution of essential mineral salts (nitrogen - N, phosphorus - P, potassium - K), to obtain maximum root growth and a maximum concentration of secondary metabolites, including bryonolic acid, without impairing the survival of the plants. By means of his general knowledge, a person skilled in the art knows how to adapt the proportions and concentrations of the various mineral salts to optimize the plant growth and particularly the root growth. The mineral salt concentrations of the nutrient solutions are, in this case, within a low electrical conductivity range advantageously extending between 0.4 to 1.6 mS/cm, preferably between 0.8 to 1.2 mS/cm, to promote a greater diversity of secondary metabolites, including bryonolic acid, within the root extract.

Advantageously, the above aeroponic culture conditions allow the obtention of some secondary metabolites, including bryonolic acid, in amount superior compared to the amount of bryonolic acid obtained in soil-culture.

According to an embodiment of the method of the present invention, the method may comprise a stimulation step of the roots. In this case, the step b) of root stimulation of said plant comprises a step of placing the roots in contact with a nitrogen-deficient stimulating nutrient solution. In this case, said solution can be a solution comprising a nitrogen proportion less than the nitrogen proportion usually considered as optimal for plant growth and in particular root growth, advantageously less than 15% of nitrogen, and more advantageously not comprising any nitrogen, said steps being sequential or simultaneous.

According to a preferred embodiment of the present invention, the method for preparing a root extract of the plant *Luffa cylindrica,* comprises the following steps:

a) a step of cultivating *Luffa cylindrica* aeroponically,
b) a step of stimulating the roots of said plant,
c) a step of solid/liquid extraction by root maceration in a solvent of the roots stimulated in step b),
d) recovery of the extract obtained in step c),
e) optionally, a step of dilution and/or clarification of the extract recovered in step d) by successive filtrations, in particular by clarifying filtration and/or sterilizing filtration.

According to an embodiment of the method of the present invention, the roots stimulation of said plant in step b) comprises a step of contacting the roots with a nitrogen-deficient nutrient solution. Placing the plants in contact with a nitrogen-deficient nutrient solution causes a "nitrogen stress" which is responsible for the stimulation of the production of secondary metabolites, particularly the production of bryonolic acid.

According to a particular embodiment of the method of the present invention, said nitrogen-deficient stimulating nutrient solution is a solution comprising less than 15% of nitrogen, preferably less than 10% of nitrogen, advantageously less than 8%, more advantageously less than 6%, less than 5%, less than 4%, less than 3%, less than 2%, less than 1% of nitrogen and even more advantageously 0% of nitrogen.

According to an embodiment of the method of the present invention, the root stimulation step b) makes it possible to significantly increase the content of secondary metabolites, including bryonolic acid, in the roots and thus to promote the flow of said metabolites going from the roots into the solvent chosen for the extraction, and to do so without total loss of viability of the plant so that it can be returned for culturing and then reused. In other words, the plant stimulation step promotes the production yield and secretion of the secondary metabolites, including bryonolic acid.

According to an embodiment of the method of the present invention, the root stimulation in step b) is performed by feeding the roots of said plants with a nitrogen-deficient N/P/K stimulating nutrient solution vaporized or misted onto the roots.

According to an embodiment of the method of the present invention, step b) is performed by spraying or macerating the roots of said plants with an N/P/K nutrient solution comprising less than 6% nitrogen, more preferably less than 3%, said solution preferably being vaporized or misted onto the roots.

According to an embodiment of the method of the present invention, step b) of stimulating the roots by feeding the roots of said plants with a nitrogen-deficient N/P/K nutrient solution vaporized onto the said roots is advantageously performed for a time of between 10 days and 8 weeks, preferably 3 weeks.

According to an embodiment of the method of the present invention, during step b), the mineral salt concentrations of the nutrient solutions are within a low electrical conductivity range advantageously extending between 0.4 to 1.2 mS/cm, preferably between 0.6 to 1.0 mS/cm, to promote a greater diversity of the secondary metabolites, including bryonolic acid within the root extract.

According to an embodiment of the method of the present invention, step b) is performed after step a).

According to an embodiment of the method of the present invention, step b) and step a) are performed simultaneously, and the nutrient solution is then substituted by the stimulating solution.

The plants cultivated and stimulated are then typically subjected to a step c) of solid/liquid extraction by root maceration under given conditions in terms of solvent, of temperature and of extraction time, so as to obtain a root extract enriched in bryonolic acid, and certainly with other secondary metabolites. Indeed, the amount of certain fatty acids

(palmitic acid, linoleic acid and $\alpha$-linolenic acid) is also increased after a thin layer chromatography (TLC) analysis of a stimulated root extract of *Luffa cylindrica* (data not shown). Solid/liquid extraction is a solvent-based extraction technique which consists in extracting one or more chemical species that are in a solid and that are soluble in said solvent. Extraction leads to the recovery, in a suitable solvent, of the secondary metabolites, including bryonolic acid, contained in the roots, by means of placing said solvent in contact with the roots, also known as the soaking step, for a suitable time. Next, said solvent containing the secondary metabolites, including bryonolic acid, released by the roots is recovered.

According to a preferred embodiment of the method of the invention, step c) of solid/liquid extraction of the roots stimulated during step b) is preceded by an additional washing step in which the solvent diffused to the plants is clear water. The supply in the extraction solvent of the elements contained in the nutrient solution or in the stimulating nutrient solution is thus limited during the step of soaking in the solvent.

According to an embodiment of the method of the present invention, the root maceration is performed on cut and dried roots.

According to another more particular embodiment of the method of the present invention, the root maceration is performed on cut and then dried and optionally milled roots. The drying method can be any suitable drying method known by a person skilled in the art, and notably by placing the roots biomass at a temperature of between 30°C and 60°C for 24 hours to 72 hours, preferably in a dry environment. Root biomass may notably be dried in a ventilated oven. The milling of the root biomass may be performed by implementing any suitable milling methods known to those skilled in the art, and notably by placing the root biomass in a ball mill or a knife mill or a hammer mill. The plants, after the step of cutting the roots, are returned to aeroponic culturing according to steps a) and optionaly b) so as to recommence their root growth and to promote the production by the roots of secondary metabolites, preferably bryonolic acid.

According to another more particular embodiment of the method of the present invention, the method comprises a step of cutting the roots and drying the cut roots, prior to the step of maceration, the maceration being performed by placing the cut dried roots in contact with a solvent.

According to another more particular embodiment of the method of the present invention, the method comprises a step of cutting the roots, of drying the cut roots and then of milling the cut dried roots, prior to the step of macerating said roots, the maceration being performed by placing the cut dried milled roots in contact with a solvent.

According to a preferred embodiment of the method of the invention, the step c) of solid/liquid extraction by root maceration comprises contacting the roots with a solvent selected from isopropyl myristate, triglycerides, tri-ethyl citrate, dicaprylyl ether, glyceryl isostearate, glyceryl stearate, ethyl acetate, vegetable oils or a mixture thereof.

Preferably, solvents can be selected from isopropyl myristate, triglycerides, tri-ethyl citrate, dicaprylyl ether, glyceryl isostearate, glyceryl stearate, ethyl acetate and vegetable oils (for example apricot kernel oil, sunflower oil, rape oil). In a preferred embodiment, the solvent is dicaprylyl ether. In a particular embodiment, the dicaprylyl ether is bio-based. More preferably, the solvent is bio-based dicaprylyl ether.

According to another preferred embodiment of the method of the invention, the solid/liquid extraction step c) comprises placing the roots in contact, for example by maceration, with dicaprylyl ether, particularly preferably bio-based dicaprylyl ether.

According to an embodiment of the method of the present invention, step c) comprises placing in the roots in contact, for example by maceration, with the solvent for a time between 30 min and 48 hours, more particularly between 1 hour and 4 hours.

According to another particular embodiment of the method of the present invention, step c) comprises placing the roots in contact, for example by maceration, with the solvent at a temperature between 20°C (room temperature) and 80°C, more particularly between 40°C and 60°C.

According to another particular embodiment of the method of the present invention, for the root maceration of dried roots, the ratio of the amount of dried roots to the amount of solvent ranges between 1 kg of dried roots/10 kg of solvent to 1 kg of dried roots/100 kg of solvent, more preferably between 1 kg of dried roots/20 kg of solvent and 1 kg of dried roots/40 kg of solvent.

In the context of the invention, the term "root maceration in solvent" preferably means a root maceration for which the solvent placed in contact with the roots is a solvent which can be, for example, isopropyl myristate, triglycerides, tri-ethyl citrate, dicaprylyl ether, glyceryl isostearate, glyceryl stearate, ethyl acetate and vegetable oils (for example apricot kernel oil, sunflower oil, rape oil), and more preferably said solvent is dicaprylyl ether.

According to an embodiment, the method according to the invention advantageously can comprise one or more additional steps of treating said plant root extract, which can be selected from:

- dilution,
- at least one filtration, in particular clarifying filtration and/or sterilizing filtration,
- solid/liquid extraction,
- purification,
- bleaching of the liquid root extract.

Such treatment methods are well known by a person skilled in the art.

Another subject-matter of the present invention refers to a root extract of the plant *Luffa cylindrica* obtained by the method as detailed above.

In the context of the present disclosure, the concentration of bryonolic acid in a root extract of plants, for example *Luffa cylindrica,* is determined by measuring the area of the peak corresponding to the bryonolic acid on the chromatogram of the analysis by liquid chromatography coupled to mass spectrometry (UPLC-MS) of said root extract. The area of the bryonolic acid peak is given to the relative area of the peak in the chromatogram of the UPLC-MS analysis of a standard solution comprising 125 mg/L of bryonolic acid. The quantification of bryonolic acid is equal to the following:

$$125 \times \frac{area\ of\ the\ bryonolic\ acid\ peak}{area\ of\ the\ bryonolic\ acid\ peak\ at\ 125\ mg/L}\ [\text{mg bryonolic acid/L}]$$

Another subject-matter of the present invention refers to a cosmetic or dermatological or nutraceutical composition comprising a root extract of the plant *Luffa cylindrica* as defined above, and optionally one or more excipients, which are preferably cosmetically or dermatologically or nutraceutically acceptable. In a preferred embodiment, the composition of the invention is a cosmetic composition comprising a root extract of the plant *Luffa cylindrica* as defined above, and optionally one or more cosmetically acceptable excipients. In another preferred embodiment, the composition of the invention is a dermatological composition comprising a root extract of the plant *Luffa cylindrica* as defined above, and optionally one or more dermatologically acceptable excipients. In another preferred embodiment, the composition of the invention is a nutraceutical composition comprising a root extract of the plant *Luffa cylindrica* as defined above, and optionally one or more nutraceutically acceptable excipients.

According to preferred embodiments of the compositions of the present invention, the bryonolic acid represents at least 10%, preferably at least 15%, at least 20%, at least 25%, at least 30%, at least 35%, at least 40%, at least 45%, at least 50%, at least 55% or at least 60% by weight, relative to the total weight of the dry extract.

According to preferred embodiments of the compositions of the invention, said root extract of the plant *Luffa cylindrica* represents 0.0001 to 15%, preferably 0.001 to 10%, more preferably 0.01 to 5% by weight, relative to the total weight of the composition.

The modes of administration, the dosages and the optimal dosage forms of the cosmetic compositions according to the invention can be determined according to the criteria generally taken into account in the establishment of a cosmetic treatment adapted to a subject such as, for example, the type of skin. Depending on the type of administration desired, the cosmetic composition according to the invention may further comprise at least one cosmetically acceptable excipient. The cosmetic composition according to the present invention may further comprise at least one adjuvant cosmetically known to those skilled in the art, chosen from thickeners, preservatives, perfumes, dyes, chemical or mineral filters, moisturizing agents, thermal waters, etc.

The cosmetic composition according to the invention may further comprise other cosmetically active agents, such as other anti-aging agents, or moisturizing agents, agents having calming, soothing or relaxing activity, agents that stimulate cutaneous microcirculation, sebo-regulating agents for the care of oily skin, cleaning or purifying agents, anti-radical agents, anti-inflammatory agents, chemical or mineral sunscreens, etc.

Suitable cosmetic excipients are those well-known by a person skilled in the art. For example, suitable cosmetic excipients may be chosen from polymers, silicone compounds, surfactants, rheology agents, humectants, penetration agents, oily components, waxes, emulsifiers, film-forming agents and perfumes, electrolytes, pH adjusters, antioxidants, preservatives, dyes, pearlescent agents, pigments and mixtures thereof.

The cosmetic composition according to the invention is advantageously intended for topical application. It may in particular be in the form a cream, a milk, a lotion, a gel, a serum, a spray, a mousse, a solution, an ointment, an emulsion, a patch or a mask.

The cosmetic composition according to the invention is intended to delay or repair the appearance of skin signs of aging (intrinsic or extrinsic), and in particular extrinsic aging, including firmness, elasticity, appearence and wrinkles. Advantageously, the composition according to the invention enables for stimulating and/or restoring skin energetic metabolism, for restoring cell viability even in fatigue cells, for inducing protective response of the skin against external agents (toxic agents, exposure to the sun, or UV, to light, stress, malnutrition), for preventing oxidative stress, more generally for preventing and/or delaying the signs of aging, and for improving the firmness, the tonicity and the elasticity of the skin.

Suitable dermatological excipients are also those well-known by a person skilled in the art. For example, suitable dermatological excipients may be identical as those intended for use in cosmetics.

Suitable nutraceutical excipients are well known by a person skilled in the art. Examples include water soluble polymers like cellulosic polymers, acrylate polymers and copolymers, polyvinylpyrrolidones, water soluble polyethylene glycols, vinyl copolymers. The nutraceutical composition may be a food supplement which comprises the root extract of *Luffa cylindrica* according to the present invention. Typically, said nutraceutical composition may be in the form of a solid,

coated or non-coated tablet, liquid, powder, soft or hard gelatin capsule, etc.

Another subject-matter of the present invention refers to a root extract of the plant *Luffa cylindrica* as defined above or a composition comprising a root extract of the plant *Luffa cylindrica* as defined above for use in stimulating and/or restoring skin energetic metabolism, restoring cell viability (even in fatigue cells), inducing protective response of the skin against external agents, preventing oxidative stress, preventing and/or delaying the signs of aging, and/or improving the firmness, tonicity and/or elasticity of the skin.

Particularly, said root extract or said compositions comprising said root extract are suitable for stimulating skin energetic metabolism by modulating the mitochondrial transporters SLC25, for upregulating intrinsic anti-oxidative systems by stimulating glutathione s-transferase theta 2, glutathione peroxidase 2 and 3, for detoxyfying peroxides by enhancing peroxiredoxin-2, for restoring efficiently cell bioenergetic profiles altered by the environmental stressors, for improving the skin structure and/or for reducing wrinkles and fines lines by the stimulation of collagen types I and IV protein expression and elastic fibers.

Another subject-matter of the present invention refers to the use of a root extract of the plant *Luffa cylindrica* as defined above or of a composition (preferably cosmetic composition) comprising a root extract of the plant *Luffa cylindrica* as above for stimulating and/or restoring skin energetic metabolism, restoring cell viability (even in fatigue cells), inducing protective response of the skin against external agents, preventing oxidative stress, preventing and/or delaying the signs of aging, and/or improving the firmness, tonicity and/or elasticity of the skin.

Another subject-matter of the present invention refers to a cosmetic skin care method for preventing or delaying the appearance of skin aging effects, said method comprises applying on at least a part of the body or face skin a cosmetic composition as described above.

Olfactory receptors (ORs) are expressed and active in various human tissues including the skin. Ectopic ORs, mainly located in the olfactory epithelium and mediate the first step in odor recognition, are expressed outside the nasal area throughout the body. These ectopic ORs may have a physiological cell functions regulation role far beyond an olfaction role. Nevertheless, most of these physiological roles remain to be elucidated.

According to the transcriptomic study (data not shown) performed on the root extract of the plant *Luffa cylindrica* as defined above, it was demonstrated that, at different concentrations of said extract as described in table 4 below, this later may regulate, in human epidermis, the transcription of several olfactory receptor gene families such as OR1, OR2, OR4, OR5, OR8, OR9, OR10, OR11, OR51, OR52 and OR56, and as most of members of each family were described to be involved in several major physiological functions such as the immune response, the inflammation, the neuronal physiology, the metabolic modulation, the wound healing and the skin stress responses. Therefore, the root extract of the plant *Luffa cylindrica* according to the present invention may be used as a global skin homeostasis guardian.

In human skin, olfactif receptors OR2AT4, OR2A4/7, OR2AG2, OR6M1, OR6V1, OR5V1, OR10A6, OR11H4, OR11A1, and OR51B5 have been identified. For example, OR2AT4, modulated by some plant extracts, is involved in the skin re-epithelialization, in the skin healing processes, and in hair growth.

It was shown that the root extract of the plant *Luffa cylindrica* as defined above regulates the olfactive receptors OR10A6 and OR2AG2, described as to be involved in stress response of skin (Duroux et al., 2020). The said extract also regulates OR2AG2 which may be involved in the skin inflammation. Moreover, it may be assumed that down-regulating the expression of OR2AG1 and OR1D2 in human keratinocytes as shown by using the said extract for OR2AG2 may lead to the regulation of skin inflammation triggered by external agents such as UV and pollutants matters. Additionnaly, OR10G7 may be involved in inflammatory skin disorders such as atopic dermatitis as it has been demonstrated to be overexpressed in undifferentiated keratinocytes isolated from atopic dermatitis. Besides, OR2AG1 and OR1D2 are also involved in airway inflammation such as asthma, and other chronic lung diseases.

As keratinocytes have an immune regulatory function, the upregulation of OR1N1 following metabolic activation as demonstrated with the root extract of the plant *Luffa cylindrica* as defined above may regulate the skin immunity. Indeed, it was shown that the said extract induces an overexpression of OR1N1.

It was also shown that the root extract of the plant *Luffa cylindrica* as defined above modulates the expression of OR52B4 which is involved in vascularization process. As as consequence, the said extract may be able to regulate skin vascular diseases such as rosacea, skin aging, and dark circles. Moreover, the said extract regulates the human skin cells metabolism for which OR8J1 and OR4D2 may be involved.

OR1L4, OR4D11 and OR5B3 belong respectively to the olfactory families of OR1, OR4 and OR5. Since the skin as the olfactory epithelium is an innervated tissue and in direct contact with volatiles particles matters, these genes may be involved in a stress response mechanism. Interestingly, it has been shown that the root extract of the plant *Luffa cylindrica* as defined above modulates in a similar manner as particulate matters the OR1L4.

The root extract of the plant *Luffa cylindrica* as defined above also upregulates the expression of OR5P3, suggesting a function of the said extract in skin's neuronal physiology. Indeed, it was suggested that OR5P3 may be involved in neurogenesis and neuro-differentiation through its interaction with a peptide derived from the Nerve Growth Factor (VGF).

Accordingly, another subject-matter of the present invention refers to the use of a root extract of the plant *Luffa*

*cylindrica* as defined above or of a composition (preferably cosmetic composition) comprising a root extract of the plant *Luffa cylindrica* as defined above in the regulation of the skin ectopic olfactory receptors (ORs) expression selected from the families of OR1, OR2, OR4, OR5, OR8, OR9, OR10, OR11, OR51, OR52 and OR56.

Particularly, the regulation of the skin ectopic olfactory receptors by the said extract, or by a composition comprising said extract may preferably for the use in:

a) the activation of the stress response mechanism of the skin wherein OR1L4, OR4D11, OR5B3, OR10A6 and OR2AG2 are involved,
b) the regulation of the skin inflammation triggered by external agents wherein OR2AG2, OR1D2 and OR10G7 are involved,
c) the improvement of vascular diseases, particluarly rosacea, skin aging, and dark circles wherein OR52B4 is involved,
d) the metabolic cell regulation wherein OR8J1 and OR4D2 are involved,
e) the skin's neuronal physiology, particularly for skin neurogenesis and skin neuro-differentiation wherein OR5P3 is involved.

However, the functional effect of the root extract of the plant *Luffa cylindrica* according to the present invention is not to be construed as being limited to the description set forth herein, and may be interpreted otherwise without scientific contradiction.

Examples

The following examples illustrate different embodiments of the present invention, but the invention is not limited to these examples.

Example 1: Studies of bryonolic acid amount in different species

Different species (listed in Table 1, obtained from commercial seeds) are chosen based on their good root yield in aeroponic system. Root samples have been harvested from plants cultured in aeroponic conditions with a defined nutrient solution of N/P/K composition corresponding to 15/10/30 and to an electrical conductivity of between 0.4 and 1.6 mS/cm for a time of 1 month, without stimulation. The content of bryonolic acid in each sample is analyzed according the following protocol: the roots are dried after cutting in a ventilated oven at 40°C for 48 hours. Then the dried roots are milled with a ball mill. For duplicate each sample, 10 mg of the dried milled root powder are macerated in 1 ml of ethanol (100%) for 30 min under stirring at room temperature (20°C). Then, extracts to be analyzed are filtered through 0.2 $\mu$m on a syringe filter before HPLC-UV-MS injection as described below in example 3.

Table 1: Content of bryonolic acid (BA) in different plant species

| The contents of bryonolic acid (BA) are indicated in Table 1 species | Provider country | BA concentration (mg/mL of ethanol) | BA concentration (mg/g of dried roots) |
|---|---|---|---|
| *Ipomea nil* | United Kingdom | 0.0 | 0.0 |
| *Phaseolus lunatus* | USA | 0.0 | 0.0 |
| *Luffa operculata* | United Kingdom | 14.7 | 1.5 |
| *Cucumis metuliferus* | France | 15.7 | 1.6 |
| *Cichorium intybus* | France | 0.0 | 0.0 |
| *Benincasa hispida* | France | 13.5 | 1.4 |
| *Coccinea grandis* | France | 0.0 | 0.0 |
| *Momordica charaptia* | United Kingdom | 0.0 | 0.0 |
| *Luffa cylindrica roots* | France | 48.0 | 4.8 |
| *Luffa cylindrica leaves* | France | 0.0 | 0.0 |

*Luffa cylindrica* is the species that produces the most bryonolic acid (3 to 4-fold) in their roots after one month in aeroponic culture system, even without stimulation. This amount is also 6 times superior than this described in Table 1 of Jai Cho et al. (Formation of bryonolic acid in cucurbitaceous plants and their cell cultures, phytochemistry, vol. 31, No. 11,

pp. 3893-3896, 1992). In accordance with the literature, this study confirms that *Luffa cylindrica's* leaves do not contain bryonolic acid.

In order to confirm the bryonolic acid concentration in root in a non-aeroponic culture, seedlings of *Luffa cylindrica* are cultivated in pots with soil in greenhouse. The content of bryonolic acid in roots is analyzed according the following protocol: the roots are washed, then dried after cutting in a ventilated oven at 40°C for 48 h. Then the dried roots are milled with a ball mill. For duplicate each sample, 12 mg of the dried milled root powder are macerated in 1.2 ml of ethanol (100%) for 60 minutes under stirring at room temperature (20°C approximatively). Then, extracts to be analyzed are centrifugated and the supernatant is pulled in a vial before HPLC-UV-MS injection as described below in example 3. Bryonolic acid in the roots cultivated with a non-aeroponic culture is detected, however its amount is under the HPLC limit of quantification. This result confirms the influence of aeroponic culture for improving the bryonolic acid content in roots of *Luffa cylindrica.*

Example 2: Comparison of bryonolic acid content in a root extract from *Luffa cylindrica* cultured in aeroponic system with or without a stimulation step Root extracts are prepared from dried roots of *Luffa cylindrica* obtained from commercial seeds (French supplier, Graines Baumaux). The seedlings are installed in aeroponic culture system. The plants in aeroponics are cultured with a nutrient medium composed by 15/10/30 respectively nitrogen/phosphorus/potassium (N/P/K) having an electrical conductivity of between 0.4 and 1.6 mS/cm for 4 weeks, the roots are cut (T0) and then plants are arranged in two separated aeroponic systems (surface of 20 $m^2$):

- for the non-stimulated conditions, the plants are maintained in culture with a nutrient medium composed by 15/10/30 (N/P/K) having an electrical conductivity of between 0.4 and 1.6 mS/cm for 6 weeks,
- for the stimulated conditions, the plants are maintained in culture with a nutrient medium composed by 0/15/40 (N/P/K) having an electrical conductivity of between 0.4 and 1.2 mS/cm for 6 weeks.

Afterwards, root samples are harvested, dried and milled: this constitutes the first cut.

After this first cut, plants are re-fed as the following:

- for the non-stimulated condition, the plants are maintained in culture with a nutrient medium composed by 15/10/30 (N/P/K) having an electrical conductivity of between 0.4 and 1.6 mS/cm for 3 weeks,
- for the stimulated condition, the plants are maintained in culture with a nutrient medium composed by 0/15/40 (N/P/K) having an electrical conductivity of between 0.4 and 1.2 mS/cm for 3 weeks.

Afterwards, root samples are harvested again, dried and milled: this constitutes the second cut.

The content of bryonolic acid in each sample is analyzed according the following protocol: the roots are dried after cutting in a ventilated oven at 40°C for 48 hours. Afterwards, dried roots are milled with a knife mill. For duplicate each sample, 100 mg of the dried milled root powder are macerated in 1,4 ml of methanol (100%) for 30 min under stirring at room temperature (20°C approximatively). Then, extracts to be analyzed are filtered through 0.45 μm on a syringe filter before HPLC-UV-MS injection described below.

The apparatus used for the analysis step is a HPLC Agilent 1200 (1260 Infinity Quaternary Pump, 1200 Series HiP-ALS $SL^+$ autosampler, 1260 Infinity Thermostatted Column Compartment, 1260 Infinity Diode Array Detector) operating in reverse phase with a Poroshell 120 (Agilent) C18 of dimensions 150 mm x 3.0 mm, 2.7 μm. The mobile phase consists of a solvent A (ultrapure Milli-Q water, Merck Millipore + 0.1% formic acid, Carlo Erba, Val-de-Reuil, France) and of a solvent B (acetonitrile, Sigma-Aldrich Chemie GmbH, Steinheim, Germany), the gradient of which was programmed in the following manner: phase B (%) 94% (0 - 14 min); 94 - 100% (14 - 15 min); 100% (15 - 20 min). The analysis flow rate is 0.4 mL/min with an oven temperature of 25°C. At the column outlet, a diode array detector records the UV spectra between 210 to 600 nm. The apparatus is coupled to a mass spectrometer (Agilent 6120 Quadrupole LC/MS) operating with electrospray ionization (4.5 kV) and atmospheric pressure chemical ionization in negative mode in an m/z range of between 100 and 1000. The software ChemStation is used to run the system.

The content of bryonolic acid and weight of dried roots are indicated in Table 2 below.

Table 2: Amount of bryonolic acid and weight of dried roots for the different cuts

| | | |
|---|---|---|
| T0 (before condition of stimulation or not) | Bryonolic acid (mg/g of dried roots) | 4.8 |
| | Weight of dried roots (g/$m^2$) | 20.75 |
| | | |

(continued)

| Condition | | Without stimulation | With stimulation |
|---|---|---|---|
| First cut | Bryonolic acid (mg/g of dried roots) | 0.70 | 1.84 |
| | Weight of dried roots (g/$m^2$) | 39 | 52 |
| Second cut | Bryonolic acid (mg/g of dried roots) | 1.03 | 7.03 |
| | weight of dried roots (g/$m^2$) | 27 | 45 |

With stimulation, the production of bryonolic acid and the production of roots (evaluated by the weight of the dried roots) are improved compared to root of the same plant in aeroponic culture system without stimulation:

- increased yield of bryonolic acid in the roots after stimulation (mg/g of dried roots): multiplied by 7 for the second cut
- increased production of root after stimulation (g/$m^2$): multiplied by 1,5 for the second cut
- total increased bryonolic acid yield (mg bryonolic acid/$m^2$): multiplied by 11

Example 3: Preparation of a root extract of *Luffa cylindrica* by means of the method according to the invention and characterization of the root extract obtained Root extracts are prepared from dried roots from *Luffa cylindrica* obtained from commercial seeds (for exemple: French supplier, Graines Baumaux). A root extract of *Luffa cylindrica* is obtained according to the following process:

a) aeroponic cultivation of the plant *Luffa cylindrica* with a defined nutrient medium of N/P/K composition corresponding respectively to 15/10/30 and to an electrical conductivity of between 0.4 and 1.6 mS/cm for a time of between 1 week and 6 weeks,
b) stimulation of the plant during 2 to 6 weeks with a nitrogen stress by using a nutrient solution of N/P/K composition comprising: less than 6% nitrogen, 15% phosphorus and 40% potassium, and with an electrical conductivity of 0.4 to 1.2 mS/cm,

b') rinsing with clear water followed by draining of the roots stimulated during step b)
b") cutting and drying the roots at a temperature between 30°C to 60°C for 24 to 72 hours in a ventilated oven

c) solid/liquid extraction by maceration of the dried *Luffa cylindrica* roots in a solution of dicaprylyl ether during 1 to 4 hours, at a temperature between 20°C to 80°C
d) recovering of the root extract obtained during step c),
e) the root extract recovered in step d) is clarified by clarifying filtration.

The root extract of *Luffa cylindrica* thus obtained has 840 mg of dry extract per kilogram of extract and contains 300 $\pm$ 10 mg of bryonolic acid per kilogram of extract, corresponding approximately to 35% of bryonolic acid by the weight of the dry extract.

The quantification of the amount of bryonolic acid in the root extracts of *Luffa cylindrica* was performed according to an approach by liquid chromatography coupled with mass spectrometry.

The apparatus used for the analysis step is a UPLC Shimadzu Nexera X2 (LC-30AD pumps, SIL-30AC sample passer, CTO-20A oven, SPD-M20A diode array detectors; Kyoto, Japan) operating in reverse phase with a Kinetex® Biphenyl column (Phenomenex®, Torrancce, CA, USA) of dimensions 150 mm x 1.1 mm, 2.6 $\mu$m. The mobile phase consists of a solvent A (ultrapure Milli-Q water, Merck Millipore + 0.1% formic acid, Carlo Erba, Val-de-Reuil, France) and of a solvent B (acetonitrile, Sigma-Aldrich Chemie GmbH, Steinheim, Germany), the gradient of which was programmed in the following manner: phase B (%) 5 - 75% (0 - 3 min); 75% (3 - 5.4 min); 75 - 90% (5.4 - 5.6 min), 90% (5.6 - 12 min), 90 - 5% (12 - 12.1 min), 5% (12.1 - 15 min). The analysis flow rate is 0.5 mL/min with an oven temperature of 40 °C. At the column outlet, a diode array detector records the UV spectra between 190 to 370 nm. The apparatus is coupled to a mass spectrometer (Shimadzu LCMS-2020) operating with electrospray ionization (4.5 kV) in negative mode in an m/z range of between 100 and 1000. The software LabSolutions is used to run the system.

The bryonolic acid quantification is performed by means of measuring the area of the peak of a bryonolic acid standard (bryonolic acid purity $\geq$ 98%; [CAS number: 24480-25-3]) prepared at a concentration of 125 mg/L in ethanol (100%).

The bryonolic acid amount expressed as mg of bryonolic acid per liter of extract is calculated according to the following formula:

$$125 \times \frac{area\ of\ the\ bryonolic\ acid\ peak}{area\ of\ the\ bryonolic\ acid\ peak\ at\ 125\ mg/L}\ [\text{mg bryonolic acid/L}]$$

Example 4: Comparison of the bryonolic acid content in the root extract relative to the total weight of the dry extract

10 g of dry root of *Luffa cylindrica* stimulated with a nutrient medium composed by 0/15/40 (N/P/K) having an electrical conductivity of between 0.4 and 1.2 mS/cm for 6 to 18 weeks, were extracted in 110 mL of ethyl lactate solvent at room temperature, with stirring, for 5 days. The mixture was filtered and the root extract is obtained in which the bryonolic acid content and dry extract were determined. The bryonolic acid represents 5.9% by weight relative to the total weight of the dry extract.

10 g of dry root of *Luffa cylindrica* stimulated with a nutrient medium composed by 0/15/40 (N/P/K) having an electrical conductivity of between 0.4 and 1.2 mS/cm for 6 to 18 weeks, were extracted in 110 mL of 1,3-propanediol solvent at 60°C, with stirring, for 5 days. After the filtration step, the obtained filtered root extract of *Luffa cylindrica* comprises bryonolic acid at 3% by weight relative to the total weight of the dry extract.

10 g of dry root of *Luffa cylindrica* stimulated with a nutrient medium composed by 0/15/40 (N/P/K) having an electrical conductivity of between 0.4 and 1.2 mS/cm for 3 to 6 weeks were extracted in 250 mL of ethanol 100 % at room temperature, with stirring, for 10 min to 5h. Différents samples are collected at 10 min, 15 min, 30 min, 1 h, 2 h, 3 h and 5 h.The different samples were filtered to obtain root extracts for which the contents in bryonolic acid and dry extract were determined. The maximal proportion of bryonolic acid obtained was 8.1 % by weight relative to the total weight of the dry extract. The minimal proportion of bryonolic acid obtained was 5.7 % by weight relative to the total weight of the dry extract.

1.2 g, 1.5 g, 2.0 g or 3.0 g of dry root of *Luffa cylindrica* stimulated with a nutrient medium composed by 0/15/40 (N/P/K) having an electrical conductivity of between 0.4 and 1.2 mS/cm for 3 to 6 weeks were extracted in 30 mL of ethanol 100 % at room temperature, with stirring, for 30 min in order to produce four extracts with the proportion of 40 g, 50 g, 66 g and 100 g of dry roots per litter of ethanol 100 %.

The different mixtures were filtered to obtain root extracts for which the contents in bryonolic acid and dry extract were determined. The maximal proportion of bryonolic acid obtained was 8.3 % by weight relative to the total weight of the dry extract, at 100 g of dry roots per liter of ethanol. The minimal proportion of bryonolic acid obtained was 7.2 % by weight relative to the total weight of the dry extract, at 40 g of dry roots per liter of ethanol.

For the following examples, the root extract of *Luffa cylindrica* enriched in bryonolic acid, hereinafter called LCRE, corresponds to the product obtained according to example 3, which has been adjusted at 145 mg of bryonolic acid per kilogram of extract

Example 5: LCRE stimulates gene expression of proteins involved in bioenergetic metabolism pathways and antioxidant metabolism (RHE model)

LCRE formulated at 0.125% and 0.5% as described in table 4, was topically applied on reconstructed human epidermis (RHE).

Table 4: Formulations comprising LCRE used in the following experimental studies

| Composition of gel cream in % | Placebo gel cream | Gel cream containing 1% LCRE | Gel cream containing 0.5% LCRE | Gel cream containing 0.125% LCRE |
|---|---|---|---|---|
| Water | 95.9 | 95.9 | 95.9 | 95.9 |
| Ammonium Acryloyldimethyltaur ate/VP Crosspolymer | 1 | 1 | 1 | 1 |
| Dicaprylyl Ether | 1 | 0 | 0.5 | 0.875 |
| LCRE in Dicaprylyl Ether | 0 | 1 | 0.5 | 0.125 |
| Citric Acid (and) Sodium Citrate | 1 | 1 | 1 | 1 |
| Phenoxyethanol (and) Methylparaben (and) Ethylparaben | 1 | 1 | 1 | 1 |
| Fragrance | 0.1 | 0.1 | 0.1 | 0.1 |

Then, the RHE tissues were cultured at the air-liquid interface in Epilife medium (Fisher Scientific, M-EPI-500-A) containing supplements and antibiotics (Gentamycin, Fisher Scientific, 15710-049). They were maintained in a humid atmosphere at 37°C with 5% $CO_2$. After 24 hours of topical treatment (2 mg/cm$^2$), total RNAs were extracted using RNeasy

Mini kit from Qiagen. Their concentrations and integrities were analyzed by spectrophotometry and capillary electrophoresis. Transcriptomic analysis was performed on Affymetrix human Clariom S arrays according to the Affymetrix user manual. Gene statistically significantly modulated by LCRE are presented in tables 5 and 6. The analysis was performed in comparison to a group treated with a placebo gel cream.

It was demonstrated that LCRE upregulates the three main energetic pathways: glycolysis, pentose phosphate pathway (PPP) and tricarboxylic acid cycle (TCA cycle also known as Krebs cycle), as shown in table 5 by the over expression of enzymes involved in these pathways. LCRE confirms in this RHE model its ability to stimulate skin energetic metabolism. In parallel, LCRE acted on mitochondrial transporters SLC25 that shuttle a variety of solutes across the mitochondrial membrane (table 5). Table 5 shows the modulation of gene expression at 0.125% LRCE while table 6 at 0.5% LCRE.

Table 5:

| Pathways/activity | Proteins involved | Fold change |
|---|---|---|
| Glycolysis | Phosphoglucose isomerase | 1.30 |
| | Phosphoglyceromutase 1 | 1.17 |
| PPP | 6-phosphogluconolactonase | 1.30 |
| TCA cycle | Succinyl-CoA-synthetase | 1.25 |
| SLC25 | SLC25A12 | 1.27 |
| Antioxidant | Gluthatione peroxidase 2 | 1.33 |
| | Gluthatione S-transferase omega 1 | 1.29 |

Modulation of gene expression analyzed by Affymetrix technology on RHE model treated with 0.125% of LCRE. Abbreviations: PPP: pentose phosphate pathway, TCA cycle: Tricarboxylic Acid cycle or Krebs cycle, SLC25: mitochondrial transporters. All analyses are done in triplicate. Statistics analysis was done in comparison to a placebo gel cream. Statistical test based on the moderated t" method implemented in the R Limma 3.26.8 (Smyth,2004), p-value<0.05.

Table 6:

| Pathways/activity | Proteins involved | Fold change | p-value |
|---|---|---|---|
| Glycolysis | Phosphoglucose isomerase | 1.31 | ** |
| | Aldolase A | 1.19 | # |
| | Aldolase B | 1.26 | * |
| | Triose phosphate isomarase | 1.24 | * |
| | Glyceraldehyde 3-phosphate dehydrogenase | 1.15 | # |
| | Phosphoglyceromutase 1 | 1.20 | * |
| | Phosphoglyceromutase 2 | 1.15 | # |
| | Enolase 1 | 1.23 | * |
| | Pyruvate kinase M | 1.15 | # |
| PPP | Transkelotase | 1.19 | * |
| | Transaldolase | 1.22 | # |
| TCA cycle | $\alpha$-ketoglutarate dehydrogenase, DLST subunit | 1.28 | # |
| | Succinyl-CoA-synthetase | 1.20 | # |
| | Fumarase | 1.18 | # |

(continued)

| Pathways/activity | Proteins involved | Fold change | p-value |
|---|---|---|---|
| SLC25 | SLC25A15 | 1.35 | * |
| | SLC25A20 | 1.30 | *** |
| | SLC25A21 | 1.22 | # |
| | SLC25A28 | 1.30 | * |
| | SLC25A31 | 1.17 | # |
| | SLC25A36 | 1.30 | # |
| | SLC25A39 | 1.15 | # |
| | SLC25A51 | 1.33 | * |
| Antioxidant | Gluthatione S-transferase theta 2 | 1.29 | # |
| | Gluthatione peroxidase 2 | 1.33 | * |
| | Gluthatione peroxidase 3 | 1.24 | # |
| | Peroxiredoxin 2 | 1.20 | * |

Modulation of gene expression analyzed by Affymetrix technology on RHE model treated with 0.5% of LCRE. Abbreviations: PPP: pentose phosphate pathway, TCA cycle: Tricarboxylic Acid cycle or Krebs cycle, SLC25: mitochondrial transporters. All analyses are done in triplicate. Statistics analysis was done in comparison to a placebo gel cream. Statistical test based on the moderated t" method implemented in the R Limma 3.26.8 (Smyth,2004). ***: p<0.005, **: p<0.01 *, p<0.05, #: p<0,1

These solutes are mainly involved in TCA cycle proceeding (*Palmieri et al. 2014 and 2016).* In table 6, the results show a strong involvement of LCRE in mitochondrial metabolism. Additionally, LCRE favors exchanges of solutes that are indispensable for glycolysis, PPP and TCA cycle. Interestingly, SLC25A12 was modulated significantly (p value<0.05, fold change 1.27). But, boosting mitochondria metabolism could produce oxidative stress as a consequence. LCRE upregulates intrinsic anti-oxidative systems. Particularly, LCRE stimulates three enzymes: glutathione s-transferase theta 2, glutathione peroxidase 2 and 3, which are responsible for protection of the cell form oxidative damages by coupling reduced glutathione to harmful compounds to be neutralized. LCRE enhances also peroxiredoxin-2, which catalyzes the reduction of hydrogen peroxide and organic hydroperoxides to water and alcohols, respectively, playing a role in cell protection against oxidative stress by detoxifying peroxides.

Example 6: LCRE stimulates the expression of proteins involved in bioenergetic metabolism pathways and antioxidant metabolism (Skin explant model)

LCRE formulated at 0.125%, 0.5% and 1%, as described in table 4, was topically applied on human skin explants. Skin explants were collected after abdominal surgery of a female donor (42 years old, caucasian, phototype III). Skin samples were stabilized in OxiProteomics® medium at 37°C, 5% of $CO_2$ for 24 hours and then treated with 2 mg/cm$^2$ of the different formulations (a group treated with LCRE-formulations and a placebo group). A third control group was added. The topical application was renewed 6 times, every 24 hours. The control group did not receive any treatment except the medium renewed every 24 hours. Eight hours after the last application, skin explants were sampled. Half of each explant were used for protein analysis. Proteins were extracted using a lysis buffer. Protein concentrations were determined using the Bradford method and quality control of the extraction was validated by high resolution SDS-PAGE. Mass spectrometry (MS) analysis was performed on a Dionex U3000 RSLC nano-LC system coupled to an Orbitrap Fusion mass spectrometer (Thermo Fisher Scientific).

It was observed that LCRE upregulates the three main energetic pathways (tables 7 and 8): glycolysis, pentose phosphate pathway (PPP) and tricarboxylic acid cycle (TCA cycle also known as Krebs cycle), as shown by the over expression of enzymes involved in these pathways. LCRE confirms in this skin explants model its ability to stimulate skin energetic metabolism already demonstrated in example 5 and also in example 7. Additionally, as observed and confirmed in example 7 below, LCRE exerts antioxidant properties. Indeed, an over expression of several antioxidant enzymes was observed.

Furthermore, in this skin explant model, it was confirmed by mass spectrometry (tables 7 and 8) but also by immunofluorescence (data not shown) the transcriptomic result, highlighting the stimulation of SLC25A12 by the formulation comprising 0.125% of LCRE. This later plays an important role in the malate-aspartate shuttle through the

mitochondrial membrane and transfers the reducing equivalents of NADPH plus $H^+$ playing an indispensable role for glycolysis *(*Palmieri, Eur J Physiol, 2004*)*. SLC25A12 is also involved in the regeneration of the antioxidant capacity of glutathione.

Table 7:

| Pathways/activity | Proteins involved | Fold change | *p*-value |
|---|---|---|---|
| PPP | 6-phosphogluconolactonase | 1.16 | ** |
| Antioxidant | Gluthatione peroxidase 3 | 1.55 | * |
| | Retinal dehydrogenase 1 | 1.32 | * |

Protein expression analyzed by mass spectrometry on skin explant model treated 7 days with a formula containing 0.5% of LCRE. Abbreviations: PPP: pentose phosphate pathway. All analyses were done in triplicate. Statistical test based on binary comparison versus placebo control group by t-test statistical analysis. **: p<0.01 *: p<0.05.

Table 8:

| Pathways/activity | Proteins involved | Fold change | *p*-value |
|---|---|---|---|
| Glycolysis | Phosphofructo-1 | 1.18 | |
| | Phosphoglycerate kinase | 1.14 | * |
| PPP | 6-phosphogluconolactonase | 1.23 | * |
| | Transketolase | 1.56 | * |
| TCA cycle | Aconitase | 1.36 | * |
| | $\alpha$-ketoglutarate dehydrogenase, DLD subunit | 1.20 | * |
| | Succinate dehydrogenase | 1.20 | ** |
| Antioxidant | Gluthatione reductase, mitochondrial OS | 1.53 | * |
| | Gluthatione S-transferase omega 1 OS | 1.50 | ** |
| | Gluthatione S-transferase LANCL1 OS | 1.33 | * |
| | Deaminated gluthatione amidase OS | 1.62 | * |
| | Retinal dehydrogenase 1 OS | 1.86 | * |
| | Superoxide dismutase [Cu-Zn] OS | 1.73 | * |

Protein expression analyzed by mass spectrometry on skin explant model treated 7 days with a formula containing 1% of LCRE. Abbreviations: PPP: pentose phosphate pathway, TCA cycle: Tricarboxylic Acid cycle or Krebs cycle, SLC25: mitochondrial transporters. All analyses were done in triplicate. Statistical test based on binary comparison versus placebo control group by t-test statistical analysis. **: p<0.01 *: p<0.05

The visualization of expression and localization of SLC25A12 (data not shown) following LCRE formuation topical application was studied by immunofluorescence on formalin fixed paraffin embedded skin sections. An HIER (Heat induced epitope retrieval) was performed using citrate buffer pH 6.0, then anti-SLC24A12 rabbit polyclonal antibody (Atlas Antibodies) was used at 1/500 followed by an Alexa 568 coupled secondary antibody. Nuclei were labelled with Hoechst 33342.

Example 7: LCRE restores dysregulated bioenergetic functions

Bioenergetic profiles of human skin fibroblasts were determined with the Seahorse XF24 flux analyser. The seahorse XF24 allows the determination of several bioenergetic parameters by measuring oxygen consumption rate (OCR):

- Basal respiration is defined as the oxygen consumption used to meet cellular ATP demand resulting from mitochondrial proton leak. This parameter shows the energetic demand of the cell under baseline conditions, which is the difference between the initial rate and the lowest respiration obtained with adding of rotenone and antimycin A.

- ATP production parameter shows the ATP produced by the mitochondria that contributes to meeting the energetic needs of the cells. This parameter is obtained experimentally by the decrease in oxygen consumption rate upon injection of the ATP synthase inhibitor oligomycin that blocks the $F_0$ subunit of the ATP synthase and therefore decreases the respiration rate reflecting the ATP production.
- Maximal respiration parameter shows the maximum rate of respiration that the cell can achieve. This parameter is obtained by adding to the experiment the uncoupler Carbonyl cyanide-4(trifluoromethoxy)phenylhydrazone, called FCCP, which collapses the proton. FCCP mimics a physiological energy demand by stimulating the respiratory chain to operate at maximum capacity, which causes rapid oxidation of substrates (sugars, fats and amino acids) to meet this metabolic challenge.
- Spare respiratory capacity reflects the cell's ability to respond to increased energy demand or under stress. This parameter can be an indicator of cell fitness and flexibility. This paramter is defined as the difference between maximal respiration and basal respiration (figure 1).

Normal human dermal fibroblasts (NHDF) isolated from abdominal skin of a donor were cultured in DMEM medium containing 10% Foetal Bovine Serum (FBS). Cells were plated at 15,000 cells-per-well density and were allowed to attach for 6 hours before adding or not (control condition) the chemical stressor 2-methyltetrahydrofuran at 9%, hereinafter called stressor, directly in culture medium. In this experiment, said stressor is used to mimic an exogenous stress, as it has been demonstrated that environmental damages and stress accumulation lead to depletion of energy metabolism, impacting overall metabolism (Reprogramming of energy metabolism as a driver of aging, Oncotarget (2016*)).*

For treatments, LCRE was also directly added in culture medium at the concentrations of 0.5% and 1%. Cells were then cultured for additional 18 hours before measurement with Agilent Seahorse XF Cell Mito Stress Test Kit according to manufacturer's instructions. Protein quantification was performed for all experimental conditions in order to normalize OCR data. OCR protein normalization ensures the accuracy and interpretation of the results. Indeed, in the experimental conditions, the cells were exposed to said stressor impacting sligltly the cell viability. Whole respiratory profiles (normalized to protein contents) are presented in figure 2 and bioenergetic parameters calculated are listed in table 9. Stressor abrogates cellular respiration in all experimental models. In the presence of the stressor, a strong decline or total inhibition in four major bioenergetic parameters was observed: basal respiration, maximal respiration, ATP production, and spare respiratory capacity. This was expected and it validates the stress model.

Table 9:

| Culture conditions | Parameters calculated from Seahorse XF cell mitochondrial stress test profiles (Based on oxygen consumption rate measurement, unit: pmol/min/μg proteins)<br>Mean ± SEM | | | |
|---|---|---|---|---|
| | Basal respiration | maximal respiration | ATP production | Spare respiratory capacity |
| Non treated | 179,27 ± 15,23 | 346,82 ± 35,06 | 136,44 ± 16,60 | 167,56 ± 20,21 |
| Stressed | 18,07 ± 20,76 | 24,11 ± 21,68 | -3,22 ± 2,06 | 6,04 ± 0,93 |
| LCRE at 0.5% | 155,20 ± 57,83 | 258,44 ± 91,42 | 92,74 ± 44,92 | 103,24 ± 33,59 |
| LCRE at 1% | 317,90 ± 36,86 | 732,66 ± 87,35 | 222,27 ± 20,07 | 414,76 ± 50,48 |

Bioenergetic parameters calculated for normal human fibroblasts in basal (non treated cells), stressed (by 2-methyltetrahydrofuran at 9%) and treated conditions (LCRE at 0.5 and at 1%).

LCRE at 0.5 and 1% can restore efficiently cell bioenergetic profiles altered by the stressor These results mean that cell treated with LCRE will be able to react effectively and meet the urgent energetic demand under stress conditions (due to the increase in maximal respiration parameter and the increase of spare respiratory capacity), leading to the restoration of ATP production. LCRE at 0.5% allows the recovery of respiratory functions similar to the control condition and return to the same respiratory level in all conditions tested. Additionally, LCRE at 1% improves the respiratory functions, which become superior to the control condition (figure 2).

Example 8: Antiaging properties of LCRE

In the skin explants model described in example 6, due to proteomic analysis, it has been found that LCRE has antiaging properties, as demonstrated by the stimulation of collagen type I protein expression (fold change x2.21, p value < 0.05). Complementary, histological and immunohistological analyses were performed on these skin explants to highlight

the antiaging properties. On formalin fixed paraffin embedded skin sections, HES staining (hematoxylin eosin saffron), modified Verhoeff's staining and immunofluorescence were performed. HES staining allows the morphological observation (figure 3) of the skin while modified Verhoeff's staining (figure 4) allows the visualization of elastic fibers content in the skin. In figure 3, it was demonstrated in a dose dependent manner, the antiaging effect of the LCRE compared to the placebo gel cream according to the table 4. Indeed, it was observed a dermis densification after topical application of LCRE. It is known that elastic fibers are responsible for skin elasticity. Unfortunately, with aging, the skin elasticity decreases, due to a slow down in elastic fibers production and an increase in elastase activity (*Imokawa et al., 2008*). It was demonstrated that LCRE promotes the production of elastic fibers. Indeed, in figure 4, the dark staining increases in a dose dependent manner as the concentration of LCRE increases, in comparison with the placebo gel cream according to table 4. The dark staining represents elastic fibers. Moreover, collagen type IV was also analyzed. Indeed, it is known that collagen type IV, a dermal epidermal junction major constituent, is also strongly degraded along with aging. The results obtained confirmed again the antiaging effect of LCRE, as it was demonstrated a dose dependent increase of collagen type IV expression following consecutive 7 days of topical application of LCRE.

In the RHE model described in example 5, it has been found that the formulation comprising LCRE at 0.5% stimulated the expression SLC35 genes family (table 10). SLC35 are also NST nucleotide sugar transporters. NSTs are antiporters responsible for transporting nucleotide sugars and PAPS into the Golgi apparatus, but also for the transport of the reaction products back to the cytosol. They are involved in the protein maturation process *(Song, Z. 2013, Ishida N., et al. 2004).* It suggests that the extract favors proteins synthesis and maturation. Interestingly in aging process, these both processes are slowed down.

Table 10:

| Pathways/activity | Proteins involved | Fold change | *p*-value |
|---|---|---|---|
| SLC35 | SLCB35B1 | 1.18 | * |
| | SLCB35B4 | 1.32 | # |
| | SLCB35E3 | 1.26 | # |

Modulation of gene expression analyzed by Affymetrix technology on RHE model treated with 0.5% of LCRE. Abreviations: SLC35: endoplasmic reticulum and Golgi apparatus transporters. Statitsical test based on the moderated t" method implemented in the R Limma 3.26.8 (Smyth,2004). *: $p<0.05$ and #: $p<0.1$. All analyses were done in triplicate.

The antiaging properties of LCRE were finally assessed on human volunteers during a double blind and vehicle controlled clinical study. The recommendations of the Declaration of Helsinki and the guidelines of the International Conference on Harmonization Good Clinical Practice were observed as applicable to a non-drug study. All volunteers provided written and informed consent.

The clinical evaluation was carried out under a dermatologist control with 20 caucasian women (age between 45 and 55 years, mean age 51.8) showing clinical signs of aging with wrinkles on the crow's feet (a grade ≥2, according to the Skin Aging Atlas vol.1, Caucasian type of R. Bazin and E. Doublet) and a lack of firmness and elasticity. Additionally, due to a questionnaire, it is confirmed that the enrolled volunteers in this study considered themselves as stressed and tired. Moreover, a questionnaire based on self-confidence questions allows us to select 9 non-confident volunteers. This subselection will permit to identify in a reliable manner if the cosmetic composition comprising LCRE, as described in table 4, helps these 9 volunteers to gain confidence. The volunteers were asked to apply on their hemifaces, twice a day and for two months, the cosmetic formulation comprising LCRE at 1% and the placebo gel cream (table 3). At days 0, 28 and 62, biometrological measurements were done with a cutometer. The cutometer allows the determination of skin biomechanical properties by the calculation of specific parameters: skin firmness (R0 parameter), skin tonicity (Ur parameter) and skin elasticity (Ue and R6 parameters). Then, with Visia CR®, Illustrative face pictures were taken.

In reference to figure 5, LCRE improves skin firmness, skin tonicity and skin elasticity. Indeed, all parameters were statistically significantly increased after 28 and 62 days of topical application of LCRE. In contrast, the placebo formula brings no improvement.

On the 9 non-self-confident volunteers enrolled in this study, 78% of them, described a more beautiful, regenerated, rested skin after 28 days of topical application of compositing comprising LCRE (56%, 67% and 67% respectively with placebo formula).

In addition, an improvement of skin wrinkles depth was observed with the composition comprising LCRE. The skin microrelief was also improved with a skin appearing smoother and firmer.

## Claims

1. A root extract of the plant *Luffa cylindrica,* wherein said root extract comprises bryonolic acid, wherein the bryonolic acid represents at least 10% by weight, relative to the total weight of the dry extract.

2. The root extract according to claim 1, wherein the bryonolic acid represents at least 15%, preferably at least 20%, at least 25%, at least 30%, at least 35%, at least 40%, at least 45%, at least 50%, at least 55% or at least 60% by weight, relative to the total weight of the dry extract.

3. The root extract according to claim 1 or 2, which is in liquid form and comprises a solvent selected from alcohols, glycols, ethyl lactate, isopropyl myristate, triglycerides, tri-ethyl citrate, dicaprylyl ether, glyceryl isostearate, glyceryl stearate, ethyl acetate, vegetable oils or a mixture thereof.

4. A method for preparing a root extract of the plant *Luffa cylindrica* according to claim 1, said method comprising the following steps:

   a) a step of cultivating *Luffa cylindrica* under soilless conditions, in particular aeroponically,
   b) a step of stimulating the roots of said plant,
   c) a step of solid/liquid extraction by root maceration of the roots stimulated in step b), said maceration comprises contacting the roots with a solvent selected from isopropyl myristate, triglycerides, tri-ethyl citrate, dicaprylyl ether, glyceryl isostearate, glyceryl stearate, ethyl acetate, vegetable oils or a mixture thereof,
   d) recovery of the extract obtained in step c), and
   e) optionally, a step of dilution and/or clarification of the extract recovered in step d) by successive filtrations, in particular by clarifying filtration and/or sterilizing filtration.

5. The method according to claim 4, said method comprising the following steps:

   a) a step of cultivating *Luffa cylindrica* aeroponically,
   b) a step of stimulating the roots of said plant,
   c) a step of solid/liquid extraction by root maceration in a solvent of the roots stimulated in step b), said maceration comprises contacting the roots with a solvent selected from isopropyl myristate, triglycerides, tri-ethyl citrate, dicaprylyl ether, glyceryl isostearate, glyceryl stearate, ethyl acetate, vegetable oils or a mixture thereof,
   d) recovery of the extract obtained in step c),
   e) optionally, a step of dilution and/or clarification of the extract recovered in step d) by successive filtrations, in particular by clarifying filtration and/or sterilizing filtration.

6. The method according to any one of the claims 4 or 5, wherein step b) of root stimulation of said plant comprises a step of contacting the roots with a nitrogen-deficient stimulating nutrient solution.

7. The method according to claim 6, wherein said nitrogen-deficient stimulating nutrient solution is a solution comprising less than 15% of nitrogen, preferably less than 10% of nitrogen, less than 8%, less than 6%, less than 5%, less than 4%, less than 3%, less than 2%, less than 1%, or 0% of nitrogen.

8. The method according to any one of the claims 4 to 7, wherein said method comprises a step of cutting the roots and drying the cut roots, prior to the step of maceration.

9. A root extract of the plant *Luffa cylindrica* obtained by the method according to any one of the claims 4 to 8.

10. Use of a root extract of the plant *Luffa cylindrica,* said root extract comprises bryonolic acid which represents at least 10% by weight, relative to the total weight of the dry extract for stimulating and/or restoring skin energetic metabolism, restoring cell viability (even in fatigue cells), inducing protective response of the skin against external agents, preventing oxidative stress, preventing and/or delaying the signs of aging.

11. Use of a root extract of the plant *Luffa cylindrica,* said root extract comprises bryonolic acid which represents at least 10% by weight, relative to the total weight of the dry extract for improving the firmness, tonicity and/or elasticity of the skin.

## Patentansprüche

1. Wurzelextrakt von der Pflanze *Luffa cylindrica,* wobei der Wurzelextrakt Bryonolsäure umfasst, wobei die Bryonolsäure wenigstens 10 Gew.-% ausmacht, bezogen auf das Gesamtgewicht des Trockenextrakts.

2. Wurzelextrakt gemäß Anspruch 1, wobei die Bryonolsäure wenigstens 15 Gew.-%, vorzugsweise wenigstens 20 Gew.-%, wenigstens 25 Gew.-%, wenigstens 30 Gew.-%, wenigstens 35 Gew.-%, wenigstens 40 Gew.-%, wenigstens 45 Gew.-%, wenigstens 50 Gew.-%, wenigstens 55 Gew.-% oder wenigstens 60 Gew.-% ausmacht, bezogen auf das Gesamtgewicht des Trockenextrakts.

3. Wurzelextrakt gemäß Anspruch 1 oder 2, der in flüssiger Form vorliegt und ein Lösungsmittel umfasst, das aus Alkoholen, Glycolen, Ethyllactat, Isopropylmyristat, Triglyceriden, Triethylcitrat, Dicaprylylether, Glycerylisostearat, Glycerylstearat, Ethylacetat, Pflanzenölen oder einem Gemisch davon ausgewählt ist.

4. Verfahren zur Herstellung eines Wurzelextrakts von der Pflanze *Luffa cylindrica* gemäß Anspruch 1, wobei das Verfahren die folgenden Schritte umfasst:

   a) einen Schritt des Kultivierens von *Luffa cylindrica* unter erdlosen Bedingungen, insbesondere aeroponisch,
   b) einen Schritt des Stimulierens der Wurzeln der Pflanze,
   c) einen Schritt der Fest-/Flüssig-Extraktion durch Wurzelmazeration der in Schritt b) stimulierten Wurzeln, wobei die Mazeration das In-Kontakt-Bringen der Wurzeln mit einem Lösungsmittel umfasst, das aus Isopropylmyristat, Triglyceriden, Triethylcitrat, Dicaprylylether, Glycerylisostearat, Glycerylstearat, Ethylacetat, Pflanzenölen oder einem Gemisch davon ausgewählt ist,
   d) die Gewinnung des in Schritt c) erhaltenen Extrakts und
   e) gegebenenfalls einen Schritt des Verdünnens und/oder Klärens des in Schritt d) gewonnenen Extrakts durch sukzessive Filtrationen, insbesondere durch Klärfiltration und/oder Sterilfiltration.

5. Verfahren gemäß Anspruch 4, wobei das Verfahren die folgenden Schritte umfasst:

   a) einen Schritt des aeroponischen Kultivierens von *Luffa cylindrica,*
   b) einen Schritt des Stimulierens der Wurzeln der Pflanze,
   c) einen Schritt der Fest-/Flüssig-Extraktion durch Wurzelmazeration der in Schritt b) stimulierten Wurzeln in einem Lösungsmittel, wobei die Mazeration das In-Kontakt-Bringen der Wurzeln mit einem Lösungsmittel umfasst, das aus Isopropylmyristat, Triglyceriden, Triethylcitrat, Dicaprylylether, Glycerylisostearat, Glycerylstearat, Ethylacetat, Pflanzenölen oder einem Gemisch davon ausgewählt ist,
   d) die Gewinnung des in Schritt c) erhaltenen Extrakts,
   e) gegebenenfalls einen Schritt des Verdünnens und/oder Klärens des in Schritt d) gewonnenen Extrakts durch sukzessive Filtrationen, insbesondere durch Klärfiltration und/oder Sterilfiltration.

6. Verfahren gemäß einem der Ansprüche 4 oder 5, wobei Schritt b) der Wurzelstimulation der Pflanze einen Schritt des In-Kontakt-Bringens der Wurzeln mit einer stickstoffarmen stimulierenden Nährstofflösung umfasst.

7. Verfahren gemäß Anspruch 6, wobei die stickstoffarme stimulierende Nährstofflösung eine Lösung ist, die weniger als 15% Stickstoff, vorzugsweise weniger als 10% Stickstoff, weniger als 8%, weniger als 6%, weniger als 5%, weniger als 4%, weniger als 3%, weniger als 2%, weniger als 1% oder 0% Stickstoff umfasst.

8. Verfahren gemäß einem der Ansprüche 4 bis 7, wobei das Verfahren vor dem Schritt des Mazerierens einen Schritt des Abschneidens der Wurzeln und des Trocknens der abgeschnittenen Wurzeln umfasst.

9. Wurzelextrakt von der Pflanze *Luffa cylindrica,* erhalten nach dem Verfahren gemäß einem der Ansprüche 4 bis 8.

10. Verwendung eines Wurzelextrakts von der Pflanze *Luffa cylindrica,* wobei der Wurzelextrakt Bryonolsäure umfasst, die wenigstens 10 Gew.-% ausmacht, bezogen auf das Gesamtgewicht des Trockenextrakts, zum Stimulieren und/oder Wiederherstellen des Energiestoffwechsels der Haut, Wiederherstellen der Zelllebensfähigkeit (auch bei erschöpften Zellen), Induktion einer Schutzreaktion der Haut gegen äußere Einflüsse, Vorbeugung von oxidativem Stress, Vorbeugung und/oder Verzögerung der Zeichen einer Alterung.

11. Verwendung eines Wurzelextrakts von der Pflanze *Luffa cylindrica,* wobei der Wurzelextrakt Bryonolsäure umfasst,

die wenigstens 10 Gew.-% ausmacht, bezogen auf das Gesamtgewicht des Trockenextrakts, zur Verbesserung der Festigkeit, Spannkraft und/oder Elastizität der Haut.

## Revendications

1. Extrait de racine de la plante Lula *cylindrica,* dans lequel ledit extrait de racine comprend de l'acide bryonolique, dans lequel l'acide bryonolique représente au moins 10 % en poids, par rapport au poids total de l'extrait sec.

2. Extrait de racine selon la revendication 1, dans lequel l'acide bryonolique représente au moins 15 %, de préférence au moins 20 %, au moins 25 %, au moins 30 %, au moins 35 %, au moins 40 %, au moins 45 %, au moins 50 %, au moins 55 % ou au moins 60 % en poids, par rapport au poids total de l'extrait sec.

3. Extrait de racine selon la revendication 1 ou 2, qui se présente sous forme liquide et comprend un solvant choisi parmi les alcools, les glycols, le lactate d'éthyle, le myristate d'isopropyle, les triglycérides, le citrate de triéthyle, l'éther dicaprylylique, l'isostéarate de glycéryle, le stéarate de glycéryle, l'acétate d'éthyle, les huiles végétales ou un mélange de ceux-ci.

4. Procédé de préparation d'un extrait de racine de la plante Lula *cylindrica* selon la revendication 1, ledit procédé comprenant les étapes suivantes :

   a) une étape de culture de Lula *cylindrica* dans des conditions hors sol, en particulier par aéroponie,
   b) une étape de stimulation des racines de ladite plante,
   c) une étape d'extraction solide/liquide par macération des racines stimulées à l'étape b), ladite macération comprenant la mise en contact des racines avec un solvant choisi parmi le myristate d'isopropyle, les triglycérides, le citrate de triéthyle, l'éther dicaprylylique, l'isostéarate de glycéryle, le stéarate de glycéryle, l'acétate d'éthyle, les huiles végétales ou un mélange de ceux- ci,
   d) la récupération de l'extrait obtenu à l'étape c), et
   e) optionnellement une étape de dilution et/ou de clarification de l'extrait récupéré à l'étape d) par des filtrations successives, en particulier par filtration clarifiante et/ou filtration stérilisante.

5. Procédé selon la revendication 4, ledit procédé comprenant les étapes suivantes :

   a) une étape de culture aérophonique de Lula *cylindrica,*
   b) une étape de stimulation des racines de ladite plante,
   c) une étape d'extraction solide/liquide par macération des racines dans un solvant des racines stimulées à l'étape b), ladite macération comprenant la mise en contact des racines avec un solvant choisi parmi myristate d'isopropyle, triglycérides, citrate de triéthyle, éther dicaprylylique, isostéarate de glycéryle, stéarate de glycéryle, acétate d'éthyle, huiles végétales ou un mélange de ceux-ci,
   d) récupération de l'extrait obtenu à l'étape c),
   e) éventuellement, une étape de dilution et/ou de clarification de l'extrait récupéré à l'étape d) par des filtrations successives, en particulier par filtration clarifiante et/ou filtration stérilisante.

6. Procédé selon l'une quelconque des revendications 4 ou 5, dans lequel l'étape b) de stimulation racinaire de ladite plante comprend une étape de mise en contact des racines avec une solution nutritive stimulante appauvrie en azote.

7. Procédé selon la revendication 6, dans lequel ladite solution nutritive stimulante appauvrie en azote est une solution comprenant moins de 15 % d'azote, de préférence moins de 10 % d'azote, moins de 8 %, moins de 6 %, moins de 5 %, moins de 4 %, moins de 3 %, moins de 2 %, moins de 1 % ou 0 % d'azote.

8. Procédé selon l'une quelconque des revendications 4 à 7, dans lequel ledit procédé comprend une étape consistant à couper les racines et à sécher les racines coupées, avant l'étape de macération.

9. Extrait de racine de la plante Lula *cylindrica* obtenu par le procédé selon l'une quelconque des revendications 4 à 8.

10. Utilisation d'un extrait de racine de la plante Lula *cylindrica,* ledit extrait de racine comprenant de l'acide bryonolique représentant au moins 10 % en poids, par rapport au poids total de l'extrait sec, pour stimuler et/ou restaurer le métabolisme énergétique de la peau, restaurer la viabilité cellulaire (même dans les cellules fatiguées), induire une

réponse protectrice de la peau contre les agents externes, prévenir le stress oxydatif, prévenir et/ou retarder les signes du vieillissement.

**11.** Utilisation d'un extrait de racine de la plante Lula *cylindrica,* ledit extrait de racine comprenant de l'acide bryonolique représentant au moins 10 % en poids, par rapport au poids total de l'extrait sec, pour améliorer la fermeté, la tonicité et/ou l'élasticité de la peau.

Figure 1:

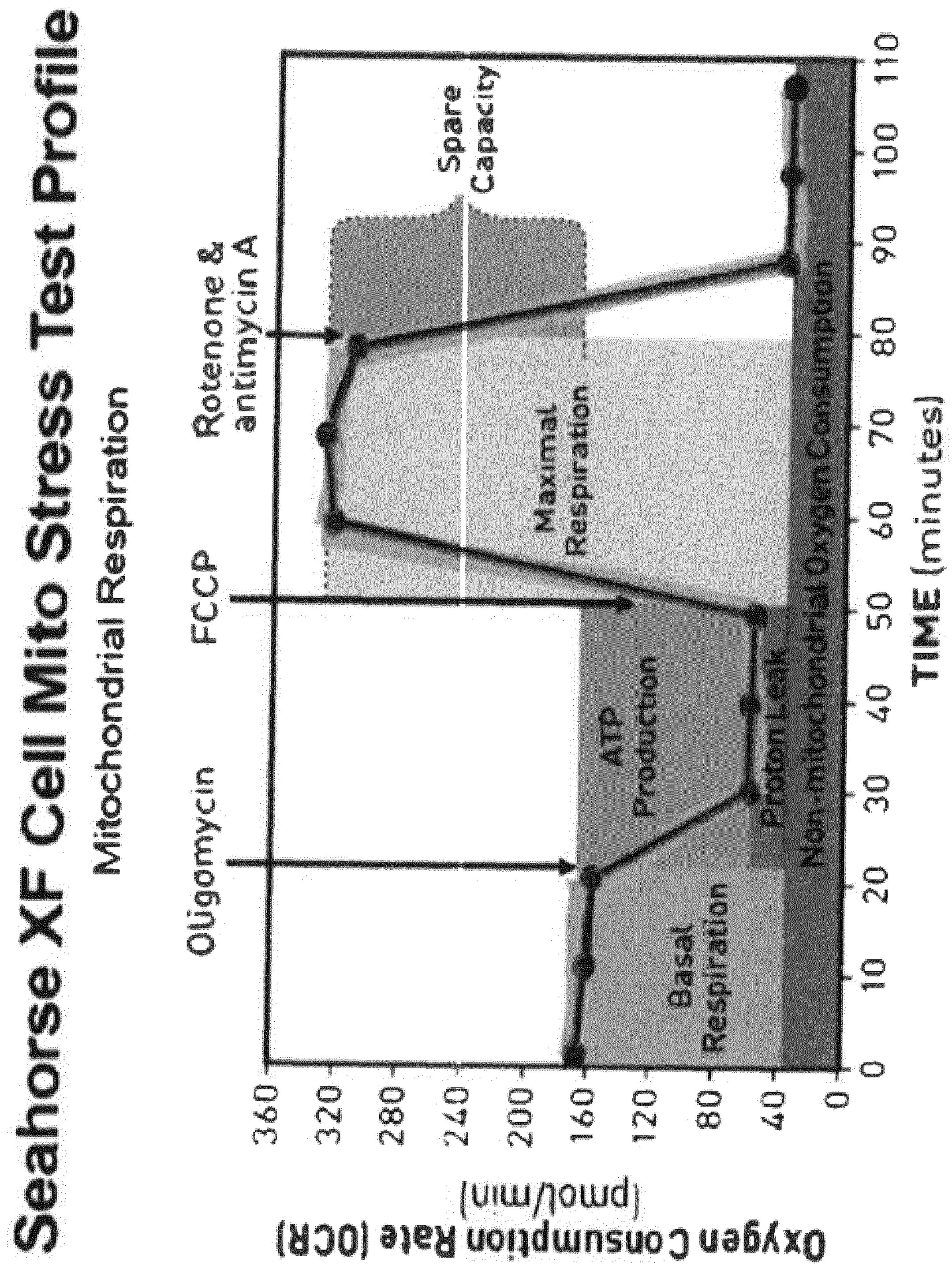
Seahorse XF Cell Mito Stress Test Profile
Mitochondrial Respiration
Oligomycin
FCCP
Rotenone & antimycin A
Spare Capacity
Maximal Respiration
ATP Production
Proton Leak
Basal Respiration
Non-mitochondrial Oxygen Consumption
Oxygen Consumption Rate (OCR) (pmol/min)
0
40
80
120
160
200
240
280
320
360
TIME (minutes)
0
10
20
30
40
50
60
70
80
90
100
110

Figures 2:

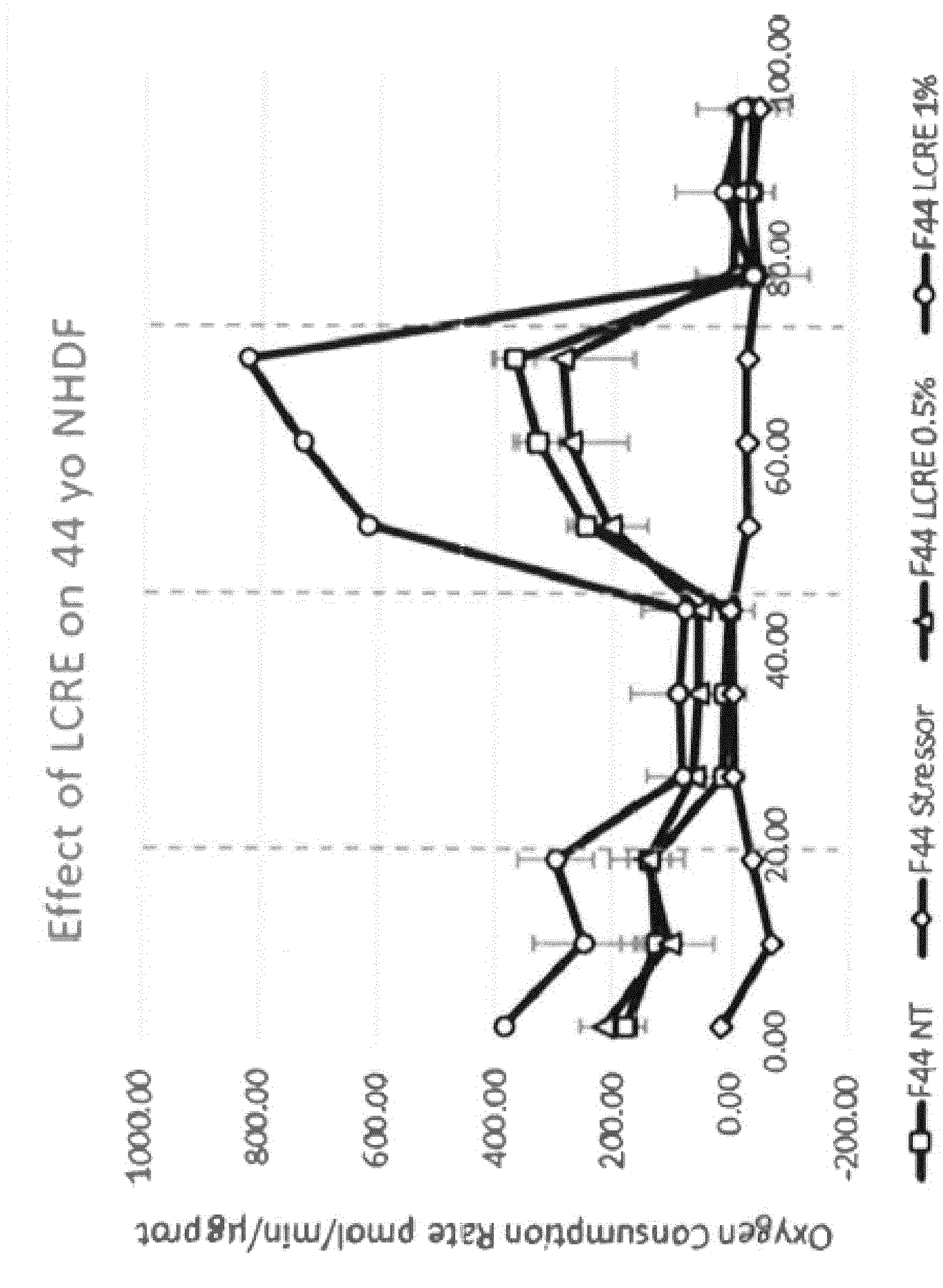
Effect of LCRE on 44 yo NHDF
Oxygen Consumption Rate pmol/min/µg prot
1000.00
800.00
600.00
400.00
200.00
0.00
-200.00
0.00
20.00
40.00
60.00
80.00
100.00
F44 NT
F44 Stressor
F44 LCRE 0.5%
F44 LCRE 1%

Figure 3:

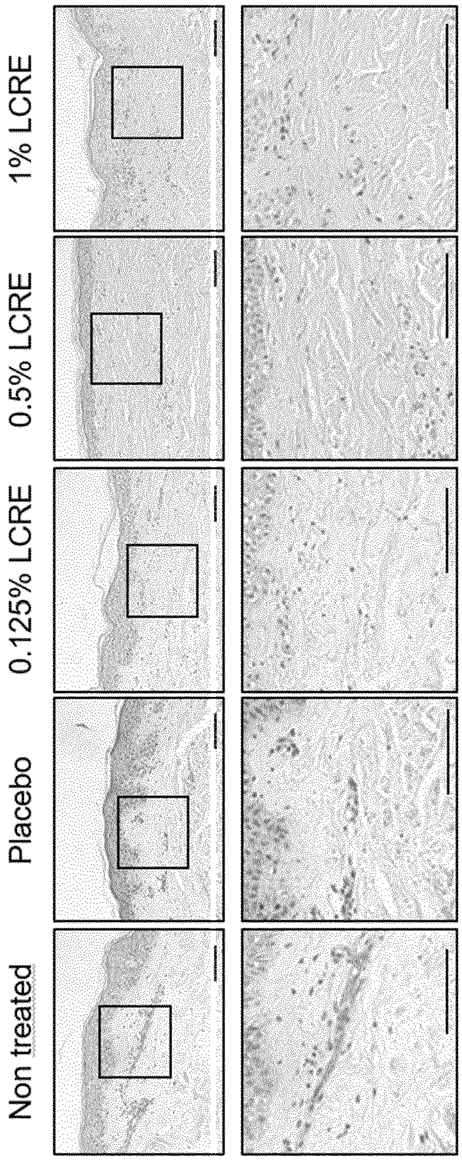

Figure 4:

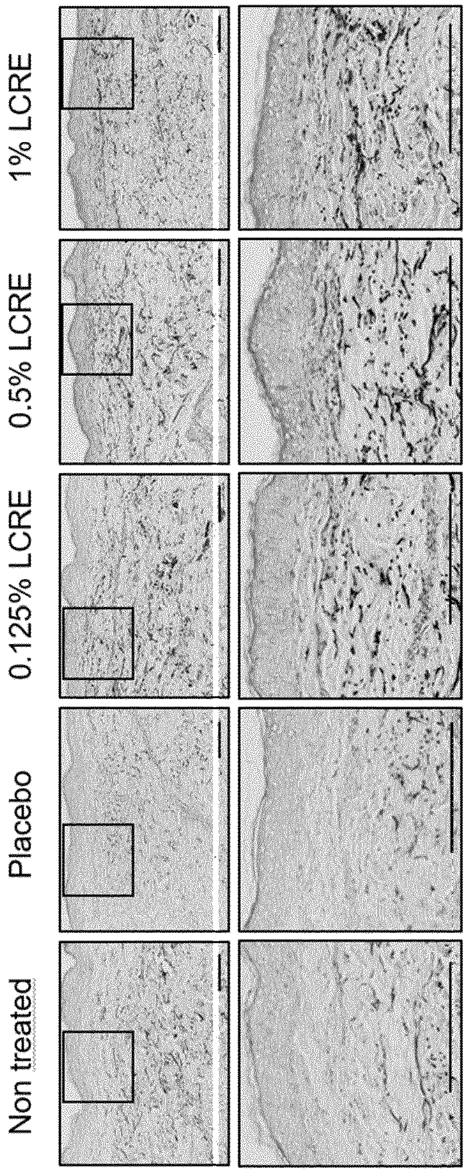

Figure 5:

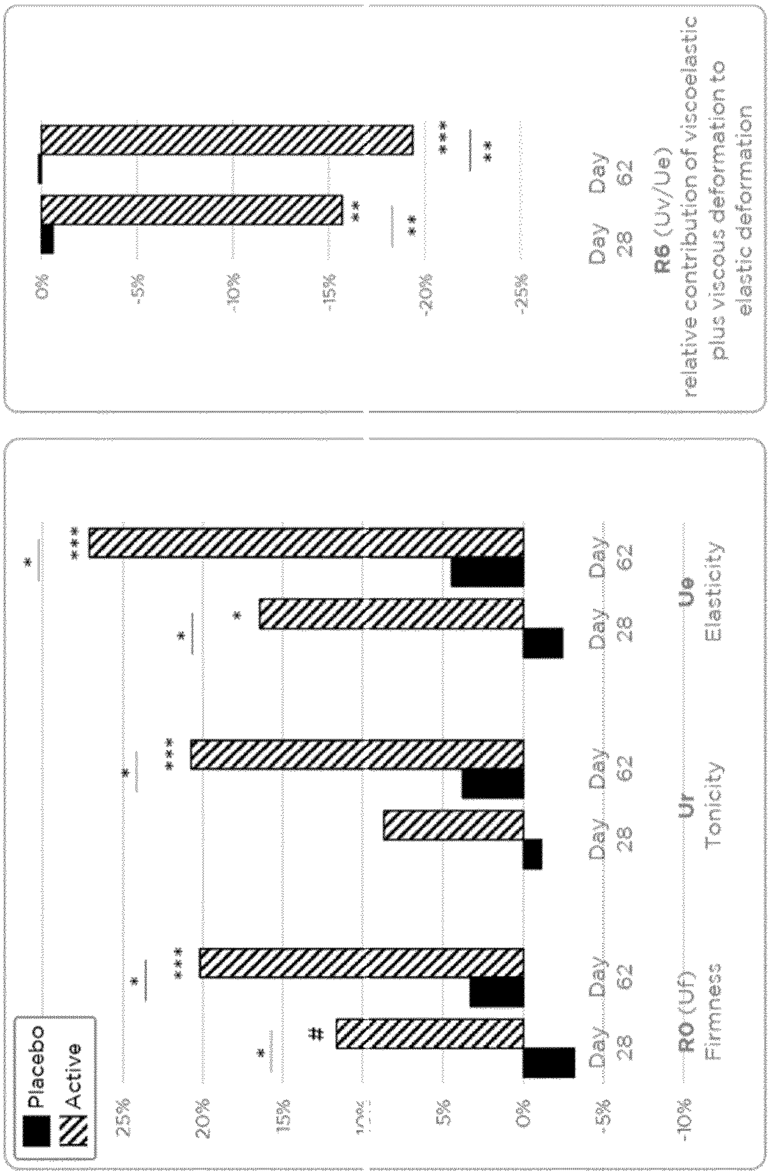
Placebo
Active
25%
20%
15%
5%
0%
-5%
-10%
Day 28
Day 62
R0 (Uf)
Firmness
Ur
Tonicity
Ue
Elasticity
0%
-5%
-10%
-15%
-20%
-25%
R6 (Uv/Ue)
relative contribution of viscoelastic
plus viscous deformation to
elastic deformation

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3659424 B
- JP 5946717 B
- EP 0359196 A2
- JP S62226912 A
- JP 2019034899 A
- JP H07109214 A
- JP 2009256271 A
- CN 107773470 A
- JP H11240823 A
- JP 4716692 B
- KR 101256180 B1
- WO 0133942 A
- WO 2018054704 A1


### Non-patent literature cited in the description


- Respiratory metabolism: glycolysis, the TCA cycle and mitochondrial electron transport. Current Opinion in Plant Biology. 2004
- Diseases caused by defects of mitochondrial carriers: A review. *Biochimica et Biophysica Acta*, 2008
- Mitochondrial transporters of the SLC25 family and associated diseases: a review. *J Inherit Metab Dis.*, July 2014, vol. 37 (4), 565-75
- Reprogramming of energy metabolism as a driver of aging. *Oncotarget*, 2016
- **LÓPEZ-OTÍN** ; **DANIEL SCHNIERTSHAUER et al.** Age-Dependent Loss of Mitochondrial Function in Epithelial Tissue Can Be Reversed by Coenzyme Q10. *J Aging Res*, 2013
- Molecular physiology and pathology of the nucleotide sugar transporter family (SLC35). *Eur J Physiol*, 2004
- **HI JAI CHO et al.** Formation of bryonolic acid in cucurbitaceous plants and their cell cultures. *phytochemistry*, 1992, vol. 31 (11), 3893-3896
- **TADAHIRO TAKEDA et al.** Bryonolic acid production in hairy roots of Trhichosanthes kirilowii Max. var. *Japonica transformed with Agrobacterium rhizogenes and its cytotoxic activity*, 1994
- **EMILY CLEGG BARKER**. The isolation and biological evaluation of anti-inflammatory and chemopreventive triterpenoid natural products. May 2015, 64-85
- **JAI CHO et al.** Formation of bryonolic acid in cucurbitaceous plants and their cell cultures. *phytochemistry*, 1992, vol. 31 (11), 3893-3896
- *CHEMICAL ABSTRACTS*, 24480-25-3
- **PALMIERI**. *Eur J Physiol*, 2004
- *Skin Aging Atlas*, vol. 1